# EUROPEAN PATENT APPLICATION

(11) **EP 4 242 242 A1**
(43) Date of publication of application: **13.09.2023**
(21) Application number: 21913231.3
(22) Date of filing: 16.09.2021
(51) Int. Cl.: C08F 230/02, C08J 9/00, B01J 31/22, B01J 31/18, C07C 253/10, C07C 255/07, C07C 255/04, B01J 35/10, C08L 43/02

(54) **POROUS POLYMER, PREPARATION METHOD THEREFOR, CATALYST AND PREPARATION METHOD FOR ADIPONITRILE**

(30) Priority: 31.12.2020 CN 202011622863
(71) Applicant: Zhejiang University, Hangzhou, Zhejiang 310058 (CN); Zhejiang NHU Company Ltd., Shaoxing, Zhejiang 312500 (CN); Shandong Nhu Amino Acid Co., Ltd., Weifang, Shandong 261108 (CN); Zhejiang NHU Special Materials Co., Ltd., Shaoxing, Zhejiang 312369 (CN)
(72) Inventor: CHEN, Zhirong, Hangzhou, Zhejiang 310058 (CN); WU, Wenbin, Shaoxing, Zhejiang 312500 (CN); YIN, Hong, Hangzhou, Zhejiang 310058 (CN); LIU, Yaqing, Shaoxing, Zhejiang 312500 (CN); ZHA, Zengshi, Shaoxing, Zhejiang 312500 (CN); FENG, Zhichao, Shaoxing, Zhejiang 312500 (CN); ZHANG, Tinglan, Shaoxing, Zhejiang 312500 (CN); HUANG, Guodong, Shaoxing, Zhejiang 312500 (CN)
(74) Representative: Hoppe, Georg Johannes
(86) International application number: PCT/CN2021/118766
(87) International publication number: WO 2022/142484

(57) **Abstract**

The present disclosure relates to a porous polymer and a method for preparing the same, a catalyst, and a method for preparing adiponitrile. The porous polymer according to the present disclosure has a pore volume of 0.3 to 2.5cm³/g; the porous polymer comprises a pore having a first pore diameter and a pore having a second pore diameter, and a ratio of a pore volume of the pore having a first pore diameter to a pore volume of the pore having a second pore diameter is 1 to 10:1, preferably 2 to 8:1, the porous polymer is obtained by self-polymerization or copolymerization of at least one of the phosphorus ligands, and a phosphorous content of the porous polymer is 1 to 5 mmol/g. The porous polymer-nickel catalyst made of the porous polymer according to the present disclosure has a significant increase in water resistance, which may reduce the consumption of phosphorus ligands, eliminating the steps of removing water from raw materials and reaction system water control, which greatly saves process equipment investment. Moreover, when it is used in the preparation of adiponitrile from butadiene, it has high catalytic activity, high reaction selectivity, and high linearity and is easy to recover and recycle.

## Description

### TECHNICAL FIELD

The present disclosure relates to a porous polymer containing a phosphorous ligand and a method for preparing the same, a porous polymer-nickel catalyst, and a method for preparing adiponitrile. Specifically, the present disclosure relates to a porous polymer formed by self-polymerization or copolymerization of at least one of the phosphorous ligands, and a catalyst formed by the coordination of the porous polymer and nickel may efficiently catalyze a hydrocyanation reaction of butadiene, an isomerization reaction of branched mononitriles, and a secondary hydrocyanation reaction of linear mononitriles to prepare adiponitrile.

### BACKGROUND

Adiponitrile (ADN) is an important organic chemical intermediate. It is a colorless and transparent oily liquid, which is slightly bitter and flammable, and the molecular formula is NC (CH₂)₄CN. Adiponitrile is mainly used in hydrogenation to produce hexamethylenediamine in industry, and then polymerized by hexamethylenediamine and adipic acid to produce polyadipamide (nylon 66). Nylon 66 has better strength, heat resistance, crystallinity, abrasion resistance, and lower water absorption compared to Nylon 6, which is widely used in the fields of automotive, mechanical industry, electronic appliances, precision instruments, and the like.

The process routes for preparing adiponitrile mainly include methods of electrolytic dimerization of acrylonitrile, catalytic ammoniation of adipic acid, and hydrocyanation of butadiene. Among them, the method of hydrocyanation of butadiene was developed based on the chlorination and cyanation process of butadiene by Du Pont, USA. This method overcomes the problems that the chlorination and cyanation process of butadiene requires large-scale matching chlor-alkali engineering, serious corrosion of equipment, and the like. Compared with other methods for preparing adiponitrile, this method has the advantages of raw materials that are readily available, low cost, low energy consumption, high product yield, and the like. Currently, the method for preparing adiponitrile from butadiene by hydrocyanation is the most advanced method for producing adiponitrile in the world.

For the production process of adiponitrile from butadiene by hydrocyanation, the key to successful industrialization is to seek a more selective and stable catalyst type and to solve the problem of catalyst recycling in the reaction system.

A variety of catalysts for hydrocyanation reactions are disclosed in prior arts.

CN1387534A discloses a catalyst used for the hydrocyanation of unsaturated organic compounds in a simple and economical way, and the catalyst uses the phosphite having the following general formula I as a ligand. Also disclosed are a transition metal complex catalyst and a method for preparing the same, as well as a use of the catalyst.

P (O-R¹)ₓ (O-R²)_{y} (O-R³)_{z} (O-R⁴), I

CN1159799A discloses a hydrocyanation process and a multidentate phosphite and nickel catalyst composition therefor. The hydrocyanation process comprises reacting an acyclic, aliphatic, monoethylenically unsaturated compound with a source of HCN in the presence of a Lewis acid, a zero-valent nickel, and a catalyst composition selected from bidentate phosphite ligands having a specific structure. The catalyst composition is mainly composed of zero-valent nickel and at least one multidentate phosphite ligand selected from the group represented by the specific structures.

WO1999052632A1 discloses a hydrocyanation reaction of olefins and an isomerization reaction of non-conjugated 2-alkyl-3-monoalkenenitriles. Also disclosed is a process for the hydrocyanation of diolefinic and olefinic compounds, comprising reacting an acyclic aliphatic diolefinic compound or an acyclic aliphatic olefin with a source of HCN in the presence of a catalyst precursor composition. The catalyst precursor composition comprises zero-valent nickel and at least one bidentate phosphorus amide ligand selected from the group having specific structures.

The complex catalysts formed by the monodentate phosphite, bidentate phosphite, or bidentate phosphoramidite ligands and zero-valent nickel described in the above patent literature are applicable to the hydrocyanation reaction of olefins. Moreover, such complex catalysts are also very suitable for the reaction system for producing adiponitrile from butadiene by hydrocyanation. However, the above catalysts are all homogeneous catalytic systems; it is difficult to separate and recover the catalysts at a later stage, thus considerably increasing the cost for recovery.

The technologies disclosed in the patent literature CN107207406A, CN1914160A, CN103189351A, CN1914154A, and CN101043946A relate to a separation process of recovering a phosphorus ligand catalyst by a method of liquid-liquid extraction, respectively. However, these processes are relatively complicated, and the organophosphorus ligands have the problem of being easily hydrolyzed. The hydrolysis of phosphorus ligands will further aggravate the difficulty of catalyst separation and recovery, so it is necessary to strictly control the water content of the reaction system. Although the raw materials need to undergo pretreatment steps such as dewatering and drying, there will still be a small amount of water during the reaction process, and the water will gradually accumulate after multiple batches of reactions, so certain water control measures should be taken during the reaction process. Therefore, a certain equipment cost will inevitably increase.

Non-patent literature ("Boosting the hydrolytic stability of phosphite ligand in hydroformylation by the construction of superhydrophobic porous framework", Yongquan Tang et al., Molecular Catalysis 474 (2019) 110408, pp. 1-6) discloses a catalyst for the hydroformylation of internal olefins. The catalyst includes Rh species and a polymer with a superhydrophobic porous framework obtained by polymerizing tris(2-tert-butyl-4-vinyl-phenyl) phosphite. The catalyst has improved durability and is easy to recover and recycle.

However, the above non-patent literature only describes the advantages of the catalyst in the hydroformylation reaction of internal olefins. The non-patent literature does not describe whether the catalyst is suitable for the hydrocyanation reaction of butadiene, or the catalytic activity, the reaction selectivity and the high linearity of the produced adiponitrile when the catalyst is applied to the hydrocyanation reaction of butadiene.

### SUMMARY

### Problem to be solved by the disclosure

In order to solve the above problems in the prior art, the present disclosure provides a porous polymer containing a phosphorus ligand and a method for preparing the same, a porous polymer-nickel catalyst, and a method for preparing adiponitrile. The porous polymer-nickel catalyst may efficiently catalyze the hydrocyanation of butadiene to prepare adiponitrile and has high catalytic activity, high reaction selectivity, and high linearity in the reaction. The porous polymer-nickel catalyst may be recovered and recycled by simple filtration and separation.

### Means for solving the problems

The present disclosure provides a porous polymer containing a phosphorous ligand, and the porous polymer has a pore volume of 0.3 to 2.5 cm³/g, preferably 0.5 to 2.0 cm³/g;

the porous polymer comprises a pore having a first pore diameter and a pore having a second pore diameter, and a ratio of a pore volume of the pore having a first pore diameter to a pore volume of the pore having a second pore diameter is 1 to 10: 1, preferably 2 to 8:1,

the pore having a first pore diameter has a pore diameter of less than 10 nm, preferably 2 to 6 nm, as measured by a nitrogen adsorption method using an NLDFT model; the pore having a second pore diameter has a pore diameter of greater than 15 nm, preferably greater than 20 nm, as measured by a nitrogen adsorption method using an NLDFT model; and

the porous polymer is obtained by self-polymerization or copolymerization of at least one of the phosphorus ligands, and a phosphorous content of the porous polymer is 1 to 5 mmol/g, preferably 1.7 to 3.9 mmol/g.

The present disclosure further provides a method for preparing the porous polymer containing a phosphorous ligand according to the present disclosure, wherein at least one of the phosphorous ligands is self-polymerized or copolymerized in the presence of a radical initiator.

The present disclosure further provides a porous polymer-nickel catalyst, comprising the porous polymer containing a phosphorous ligand according the present disclosure and a zero-valent nickel, and the content of the zero-valent nickel is 0.1 to 2 mmol/g with respect to an amount of the catalyst.

The present disclosure further provides a method for preparing adiponitrile, wherein the method comprises: sequentially subjecting to a primary hydrocyanation reaction of butadiene, an isomerization reaction of branched mononitriles, and a secondary hydrocyanation reaction of linear mononitriles in the presence of the porous polymer-nickel catalyst according to the present disclosure.

### Effect of the disclosure

The porous polymer containing a phosphorous ligand according to the present disclosure has superhydrophobicity. The porous polymer-nickel catalyst formed from this porous polymer and nickel has a significant increase in water resistance, which may reduce the consumption of phosphorus ligands, eliminating the steps of removing water from raw materials and reaction system water control, which greatly saves process equipment investment.

When the porous polymer-nickel catalyst according to the present disclosure is used in the primary hydrocyanation reaction of butadiene, the isomerization reaction of branched mononitriles, and the secondary hydrocyanation reaction of linear mononitriles, it has high catalytic activity and high reaction selectivity and is easy to recover and recycle. The linearity of the primary hydrocyanation of butadiene and the secondary hydrocyanation of linear mononitriles may reach 87.5% or more. Due to the realization of high linearity, the amount of branched mononitrile products may be greatly reduced from the aspect of the process, thereby significantly reducing equipment investment and separation energy costs.

The porous polymer-nickel catalyst provided by the present disclosure is a heterogeneous catalyst with high resistance to hydrolysis. The catalyst may be recycled in the reaction system through simple separation, and the catalyst separation and recovery steps are simplified. The number of catalyst recycling is greatly increased and the catalyst cost is reduced.

### DETAILED DESCRIPTION

Embodiments of the present disclosure are described in further detail below.

For the porous polymer containing a phosphorous ligand provided according to the present disclosure, the porous polymer comprises a pore having a first pore diameter and a pore having a second pore diameter, and a ratio of a pore volume of the pore having a first pore diameter to a pore volume of the pore having a second pore diameter is 1 to 10:1, preferably 2 to 8:1,
the pore having a first pore diameter has a pore diameter of less than 10 nm, preferably 2 to 6 nm, as measured by a nitrogen adsorption method using an NLDFT model; the pore having a second pore diameter has a pore diameter of greater than 15 nm, preferably greater than 20 nm, as measured by a nitrogen adsorption method using an NLDFT model; and
the porous polymer is obtained by self-polymerization or copolymerization of at least one of the phosphorus ligands, and a phosphorous content of the porous polymer is 1 to 5 mmol/g, preferably 1.7 to 3.9 mmol/g.

The smaller pore diameter (< 10 nm, preferably 2 to 6 nm) of the porous polymer containing a phosphorous ligand according to the present disclosure is advantageous to increasing the pore volume and specific surface area. When the porous polymer is subjected to the hydrocyanation reaction, the spatial structure of the catalyst may be changed by the confinement of micropores, and the linearity of the product may be increased. The larger pore diameter (>15 nm, preferably greater than 20 nm) of the porous polymer is advantageous to the transport and internal diffusion of reactants and products, improving the utilization of phosphorus atoms, increasing the reaction speed, and improving the activity of the catalyst. The catalyst formed of the porous polymer containing a phosphorous ligand according to the present disclosure and nickel is especially advantageous to the preparation of linear adiponitrile, which may increase the linearity of the product and has high catalytic activity. Thus, the pore volume distribution of the porous polymer containing a phosphorous ligand of the present disclosure should be such that the ratio of the pore volume of pores of < 10 nm to the pore volume of pores of > 15 nm is 1 to 10:1, preferably 2 to 8:1.

Moreover, in a preferred case, the BET specific surface area of the porous polymer of the present disclosure is 100 to 2000 m²/g, and preferably 500 to 1700 m²/g.

For the porous polymer containing a phosphorous ligand according to the present disclosure, when the pore volume of the porous polymer is within the above range, it is able to be beneficial to provide a reaction site and to achieve a balance between the in and out of reactants and products.

In addition, when the BET specific surface area of the porous polymer of the present disclosure is within the above range, there are more catalytic sites per unit volume, which is more conducive to improving the overall catalytic effect.

For the porous polymer containing a phosphorous ligand according to the present disclosure, the phosphorous ligand is represented by the following general formula (1): 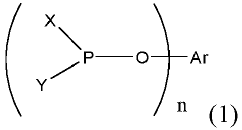
wherein: n = 1 to 4;
Ar represents a group having a substituted aromatic ring structure;
X and Y are the same or different, and each independently represents an aryloxy group or a nitrogen-containing heterocyclic group, and X and Y may form a ring via a single bond or a methylene group.

In general formula (1): when n is 1, the phosphorous ligand is a monodentate phosphorous ligand, and both X and Y are 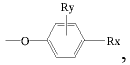 Ar is 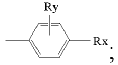 when n is 2 to 4, the phosphorous ligand is a multidentate phosphorous ligand, X and Y are the same or different, and each independently represents 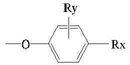 or a nitrogen-containing heterocyclic group; Ar is 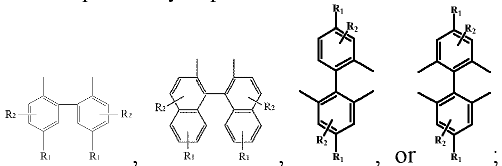 provided that, when both X and Y represent 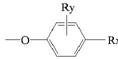 X and Y may not form a ring; when both X and Y represent a nitrogen-containing heterocyclic group, X and Y may not form a ring or form a ring via a single bond or a methylene group; and when X is 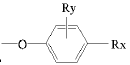 and Y is a nitrogen-containing heterocyclic group, X and Y form a ring via a methylene group; the nitrogen-containing heterocyclic group is

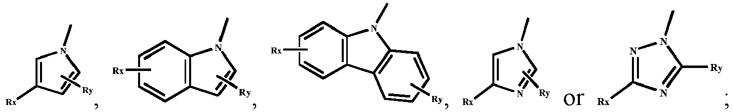

among the above, R₁ is selected from the group consisting of a hydrogen atom, a vinyl group, a propenyl group, an acryloyl group, an acrylate group, or a methacryloyl group;
R₂ is selected from the group consisting of a hydrogen atom, a halogen atom, a nitrile group, an C₁-C₁₀ alkyl group, an C₁-C₁₀ alkoxy group, an C₁-C₁₀ alkanoyl group, an C₁-C₁₀ ester group, or an C₁-C₁₀ sulfonate group;
Rₓ is selected from the group consisting of a hydrogen atom, a vinyl group, a propenyl group, an acryloyl group, an acrylate group, or a methacryloyl group;
R_{y} is selected from the group consisting of a hydrogen atom, a halogen atom, a nitrile group, an C₁-C₁₀ alkyl group, an C₁-C₁₀ alkoxy group, an C₁-C₁₀ alkanoyl group, an C₁-C₁₀ ester group, or an C₁-C₁₀ sulfonate group.

In a preferred case, the phosphorous ligand is selected from the compounds having the following structural formulae (2) to (18): 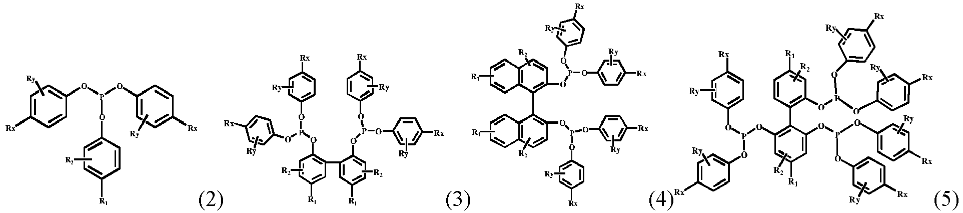 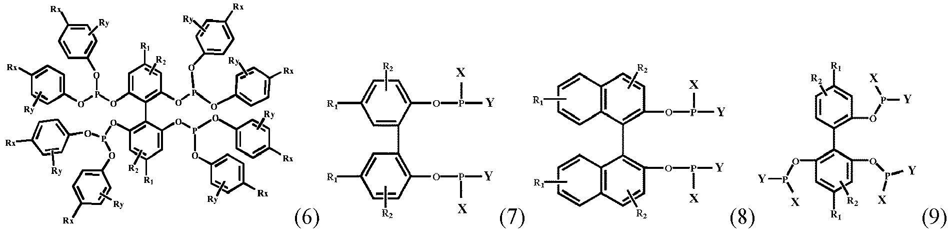 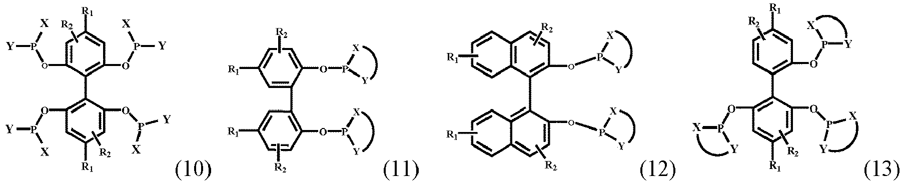 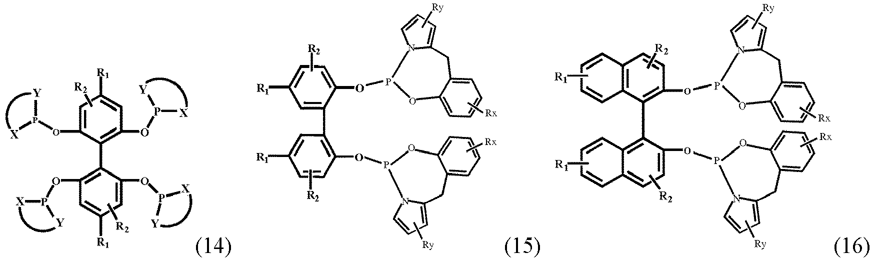 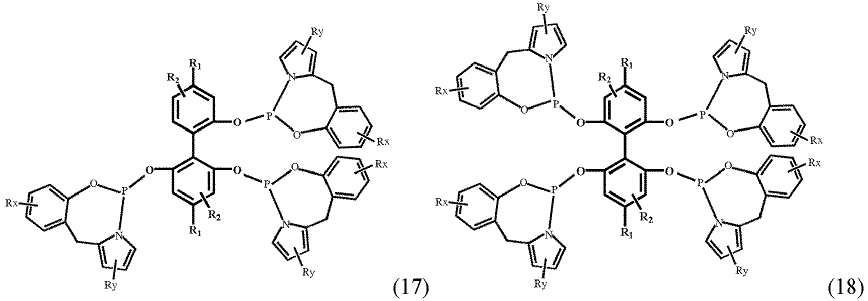 wherein R₁, R₂, Rₓ, and R_{y} are as defined in general formula (1); X and Y are the same, which both represent a nitrogen-containing heterocyclic group.

In a preferred case, among the phosphorous ligands, both X and Y are nitrogen-containing heterocyclic groups, and a structural moiety at which X and Y form a ring via a single bond or a methylene group is selected from any one of the group consisting of the following:

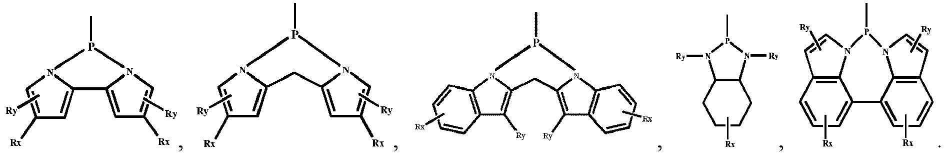

According to one embodiment of the present disclosure, the porous polymer is obtained by self-polymerization of any one of the phosphorus ligands.

According to another embodiment of the present disclosure, the porous polymer is a random copolymer obtained by copolymerization of any two of the phosphorus ligands, and a molar ratio between the two phosphorous ligands is 0.01 to 3:1, and preferably 0.05 to 2.5:1.

According to yet another embodiment of the present disclosure, wherein the porous polymer is a random copolymer obtained by copolymerization of any three or more of the phosphorous ligands.

The combination of monomers of the porous polymer containing a phosphorous ligand according to the present disclosure may be divided into the following groups:
1. The porous polymer is a homopolymer or copolymer containing monomer units derived from the monodentate phosphorous ligand represented by formula (2), and examples thereof are given below: homopolymers of formula (2); and copolymers of formulae (2) and (3), of formulae (2) and (4), of formulae (2) and (5), of formulae (2) and (6), of formulae (2) and (7), of formulae (2) and (8), of formulae (2) and (9), of formulae (2) and (10), of formulae (2) and (11), of formulae (2) and (12), of formulae (2) and (13), of formulae (2) and (14), of formulae (2) and (15), of formulae (2) and (16), of formulae (2) and (17), of formulae (2) and (18); and copolymers of three or more phosphorus ligands.
2. The porous polymer is a homopolymer or copolymer containing monomer units derived from the multidentate phosphorous ligand represented by formula (3), and examples thereof are given below: homopolymers of formula (3); and copolymers of formulae (3) and (4), of formulae (3) and (5), of formulae (3) and (6), of formulae (3) and (7), of formulae (3) and (8), of formulae (3) and (9), of formulae (3) and (10), of formulae (3) and (11), of formulae (3) and (12), of formulae (3) and (13), of formulae (3) and (14), of formulae (3) and (15), of formulae (3) and (16), of formulae (3) and (17), of formulae (3) and (18); and copolymers of three or more phosphorus ligands.
3. The porous polymer is a homopolymer or copolymer containing monomer units derived from the multidentate phosphorous ligand represented by formula (4), and examples thereof are given below: homopolymers of formula (4); and copolymers of formulae (4) and (5), of formulae (4) and (6), of formulae (4) and (7), of formulae (4) and (8), of formulae (4) and (9), of formulae (4) and (10), of formulae (4) and (11), of formulae (4) and (12), of formulae (4) and (13), of formulae (4) and (14), of formulae (4) and (15), of formulae (4) and (16), of formulae (4) and (17), of formulae (4) and (18); and copolymers of three or more phosphorus ligands.
4. The porous polymer is a homopolymer or copolymer containing monomer units derived from the multidentate phosphorous ligand represented by formula (5), and examples thereof are given below: homopolymers of formula (5); and copolymers of formulae (5) and (6), of formulae (5) and (7), of formulae (5) and (8), of formulae (5) and (9), of formulae (5) and (10), of formulae (5) and (11), of formulae (5) and (12), of formulae (5) and (13), of formulae (5) and (14), of formulae (5) and (15), of formulae (5) and (16), of formulae (5) and (17), of formulae (5) and (18); and copolymers of three or more phosphorus ligands.
5. The porous polymer is a homopolymer or copolymer containing monomer units derived from the multidentate phosphorous ligand represented by formula (6), and examples thereof are given below: homopolymers of formula (6); and copolymers of formulae (6) and (7), of formulae (6) and (8), of formulae (6) and (9), of formulae (6) and (10), of formulae (6) and (11), of formulae (6) and (12), of formulae (6) and (13), of formulae (6) and (14), of formulae (6) and (15), of formulae (6) and (16), of formulae (6) and (17), of formulae (6) and (18); and copolymers of three or more phosphorus ligands.
6. The porous polymer is a homopolymer or copolymer containing monomer units derived from the multidentate phosphorous ligand represented by formula (7), and examples thereof are given below: homopolymers of formula (7); and copolymers of formulae (7) and (8), of formulae (7) and (9), of formulae (7) and (10), of formulae (7) and (11), of formulae (7) and (12), of formulae (7) and (13), of formulae (7) and (14), of formulae (7) and (15), of formulae (7) and (16), of formulae (7) and (17), of formulae (7) and (18); and copolymers of three or more phosphorus ligands.
7. The porous polymer is a homopolymer or copolymer containing monomer units derived from the multidentate phosphorous ligand represented by formula (8), and examples thereof are given below: homopolymers of formula (8); and copolymers of formulae (8) and (9), of formulae (8) and (10), of formulae (8) and (11), of formulae (8) and (12), of formulae (8) and (13), of formulae (8) and (14), of formulae (8) and (15), of formulae (8) and (16), of formulae (8) and (17), of formulae (8) and (18); and copolymers of three or more phosphorus ligands.
8. The porous polymer is a homopolymer or copolymer containing monomer units derived from the multidentate phosphorous ligand represented by formula (9), and examples thereof are given below: homopolymers of formula (9); and copolymers of formulae (9) and (10), of formulae (9) and (11), of formulae (9) and (12), of formulae (9) and (13), of formulae (9) and (14), of formulae (9) and (15), of formulae (9) and (16), of formulae (9) and (17), of formulae (9) and (18); and copolymers of three or more phosphorus ligands.
9. The porous polymer is a homopolymer or copolymer containing monomer units derived from the multidentate phosphorous ligand represented by formula (10), and examples thereof are given below: homopolymers of formula (10); and copolymers of formulae (10) and (11), of formulae (10) and (12), of formulae (10) and (13), of formulae (10) and (14), of formulae (10) and (15), of formulae (10) and (16), of formulae (10) and (17), of formulae (10) and (18); and copolymers of three or more phosphorus ligands.
10. The porous polymer is a homopolymer or copolymer containing monomer units derived from the multidentate phosphorous ligand represented by formula (11), and examples thereof are given below: homopolymers of formula (11); and copolymers of formulae (11) and (12), of formulae (11) and (13), of formulae (11) and (14), of formulae (11) and (15), of formulae (11) and (16), of formulae (11) and (17), of formulae (11) and (18); and copolymers of three or more phosphorus ligands.
11. The porous polymer is a homopolymer or copolymer containing monomer units derived from the multidentate phosphorous ligand represented by formula (12), and examples thereof are given below: homopolymers of formula (12); and copolymers of formulae (12) and (13), of formulae (12) and (14), of formulae (12) and (15), of formulae (12) and (16), of formulae (12) and (17), of formulae (12) and (18); and copolymers of three or more phosphorus ligands.
12. The porous polymer is a homopolymer or copolymer containing monomer units derived from the multidentate phosphorous ligand represented by formula (13), and examples thereof are given below: homopolymers of formulae (13); and copolymers of formulae (13) and (14), of formulae (13) and (15), of formula (13) and (16), of formulae (13) and (17), of formulae (13) and (18); and copolymers of three or more phosphorus ligands.
13. The porous polymer is a homopolymer or copolymer containing monomer units derived from the multidentate phosphorous ligand represented by formula (14), and examples thereof are given below: homopolymers of formulae (14); and copolymers of formulae (14) and (15), of formulae (14) and (16), of formula (14) and (17), of formulae (14) and (18); and copolymers of three or more phosphorus ligands.
14. The porous polymer is a homopolymer or copolymer containing monomer units derived from the multidentate phosphorous ligand represented by formula (15), and examples thereof are given below: homopolymers of formula (15); and copolymers of formulae (15) and (16), of formulae (15) and (17), of formulae (15) and (18); and copolymers of three or more phosphorus ligands.
15. The porous polymer is a homopolymer or copolymer containing monomer units derived from the multidentate phosphorous ligand represented by formula (16), and examples thereof are given below: homopolymers of formula (16); and copolymers of formulae (16) and (17), of formulae (16) and (18); and copolymers of three or more phosphorus ligands.
16. The porous polymer is a homopolymer or copolymer containing monomer units derived from the multidentate phosphorous ligand represented by formula (17), and examples thereof are given below: homopolymers of formulae (17); and copolymers of formulae (17) and (18); and copolymers of three or more phosphorus ligands.
17. The porous polymer is a homopolymer or copolymer containing monomer units derived from the multidentate phosphorous ligand represented by formula (18), and examples thereof are given below: homopolymers of formulae (18).

In the groups 1 to 17 described above, various combinations of phosphorus ligands are only exemplarily listed, but the combinations of monomer units in the porous polymer of the present disclosure are not limited to those listed above, and all combinations of one, two, three, four or more kinds of monomer units are within the scope of the present disclosure.

Among the porous polymers with a variety of combinations described above, the ratio of the specific surface area to the pore volume of the secondary pore channels, the charge on the phosphorus atom in the phosphorous ligand, and the steric hindrance of the phosphorus ligand are the main factors affecting the reactivity and linearity selectivity. According to the evaluation results of practical application, a porous polymer formed of a phosphorus ligand containing a nitrogen-containing multidentate phosphorous ligand is preferred.

The specific phosphorus ligand monomers in the porous polymers of the groups 1 to 17 described above are shown in Table 1 below.

| **Table 1** | | | | | |
|---|---|---|---|---|---|
| | a | b | c | d | e |
| 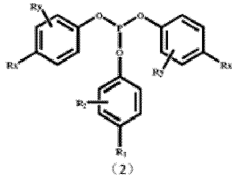 | 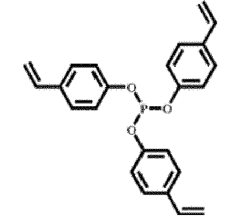 | 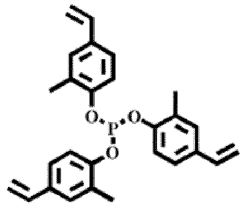 | 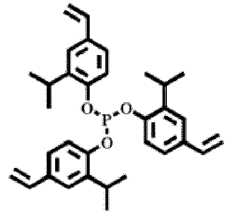 | 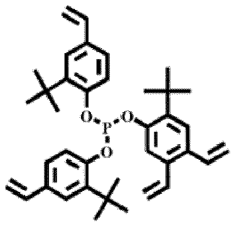 | 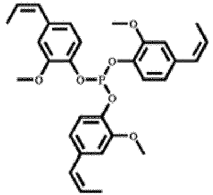 |
| 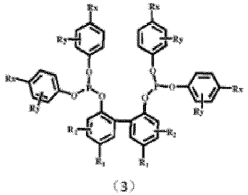 | 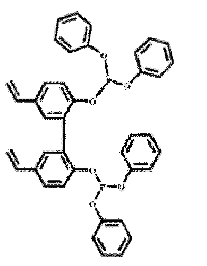 | 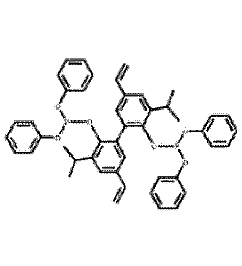 | 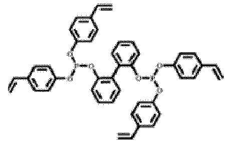 | 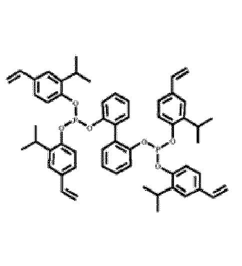 | 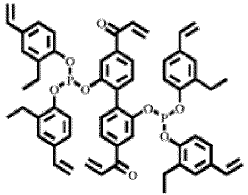 |
| 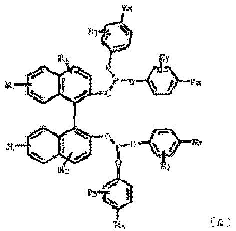 | 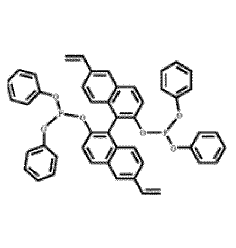 | 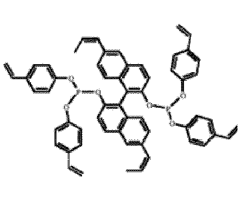 | 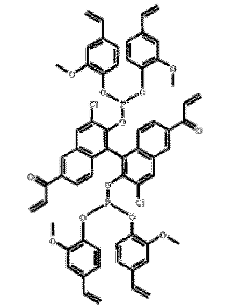 | 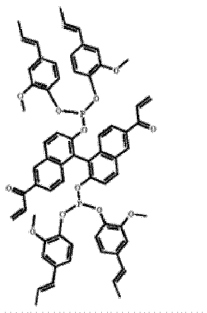 | 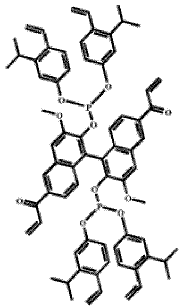 |

| | a | b | c | d | e |
|---|---|---|---|---|---|
| 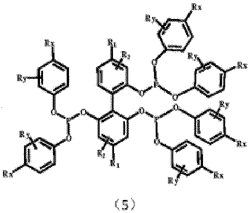 | 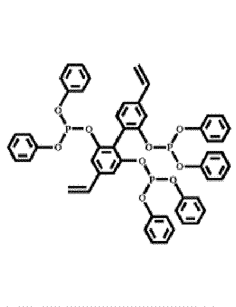 | 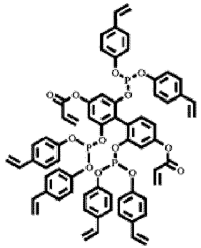 | 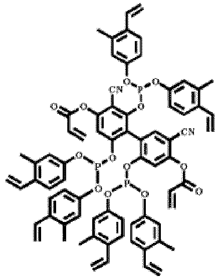 | 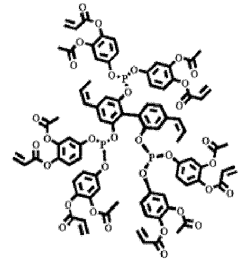 | 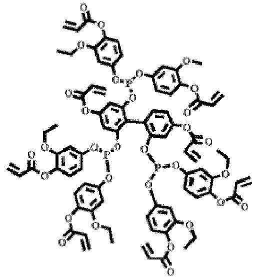 |
| 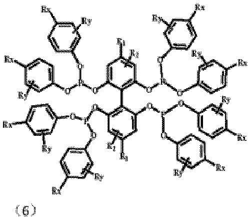 | 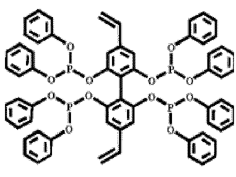 | 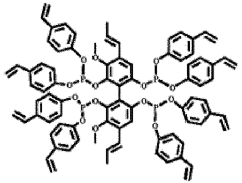 | 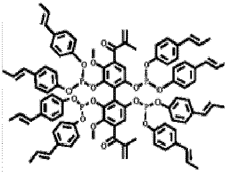 | 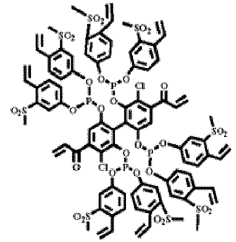 | 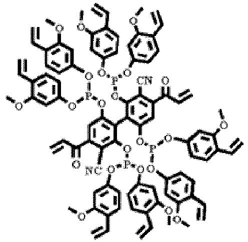 |
| 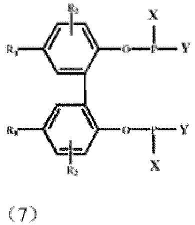 | 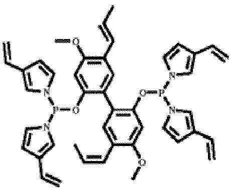 | 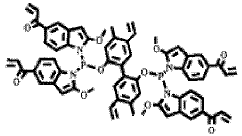 | 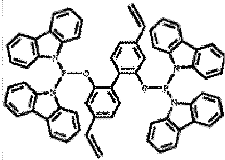 | 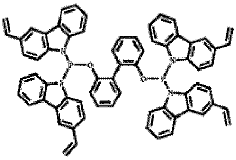 | 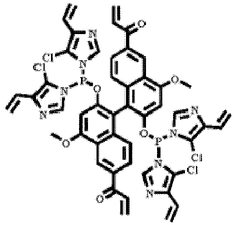 |
| 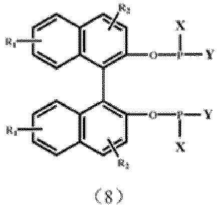 | 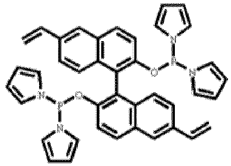 | 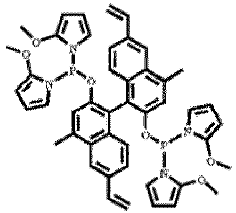 | 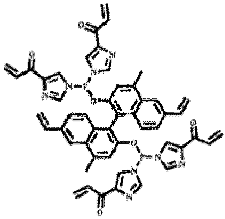 | 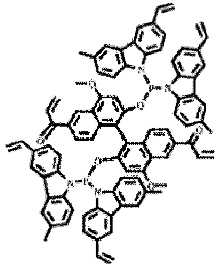 | 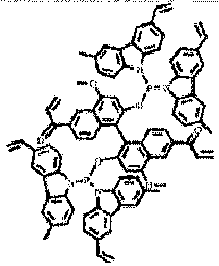 |
| 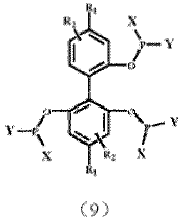 | 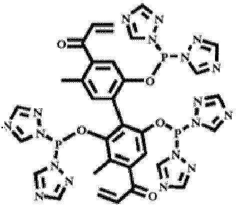 | 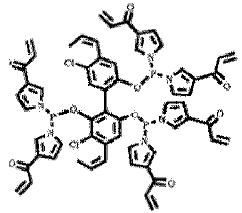 | 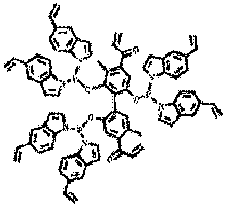 | 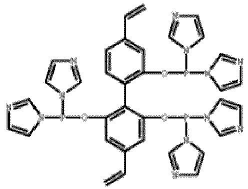 | 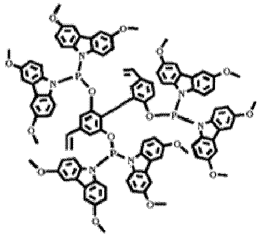 |
| 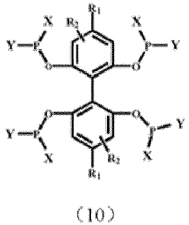 | 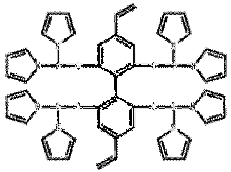 | 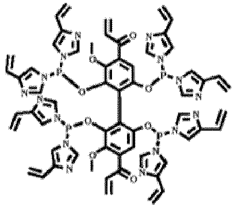 | 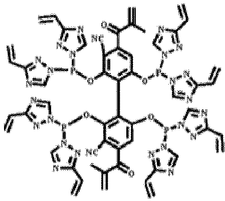 | 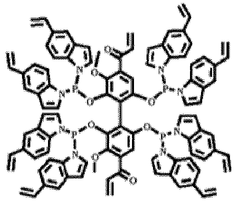 | 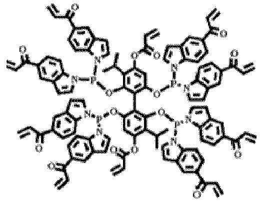 |
| 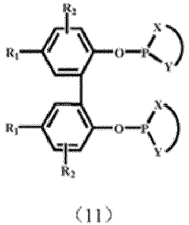 | 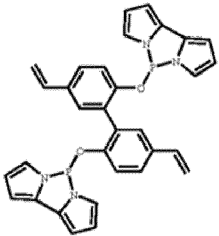 | 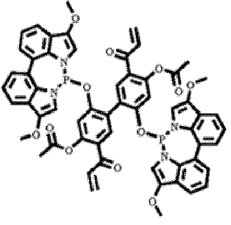 | 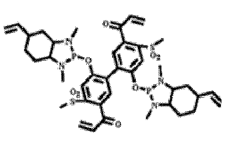 | 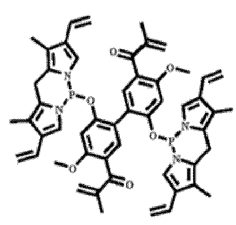 | 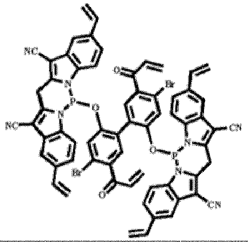 |
| 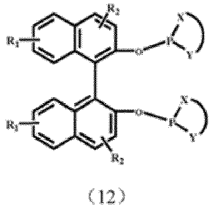 | 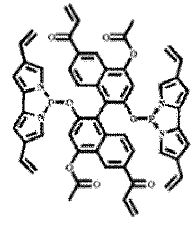 | 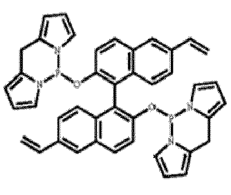 | 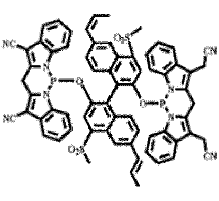 | 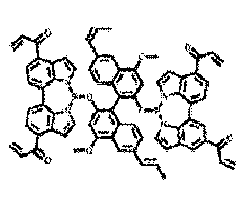 | 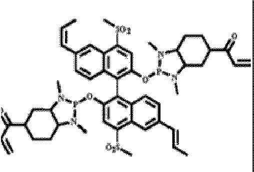 |
| 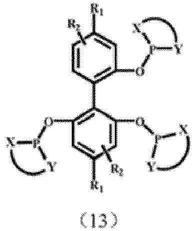 | 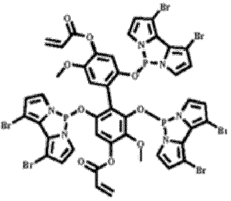 | 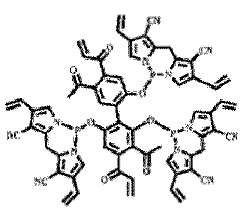 | 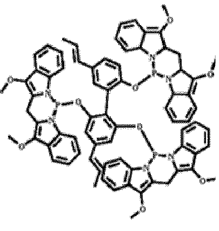 | 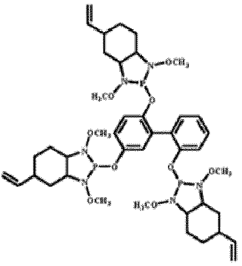 | 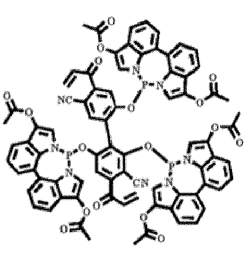 |
| 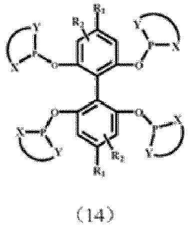 | 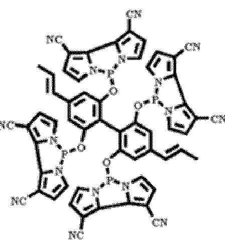 | 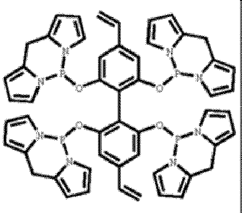 | 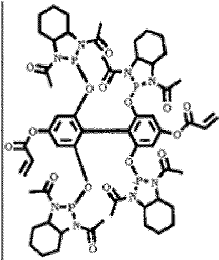 | 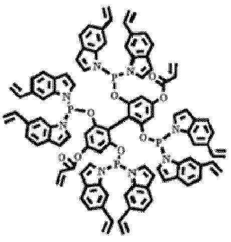 | 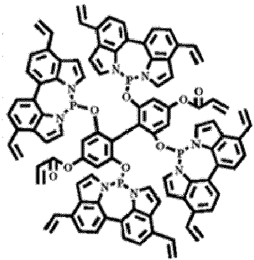 |
| 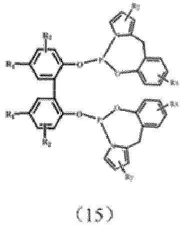 | 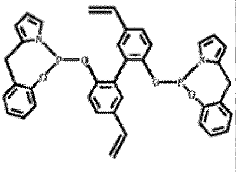 | 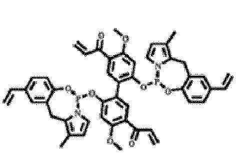 | 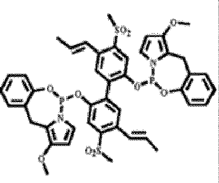 | 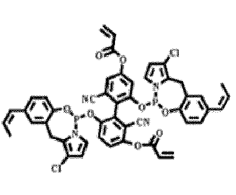 | 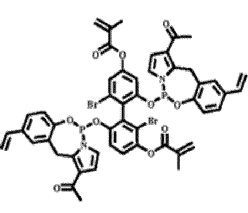 |
| 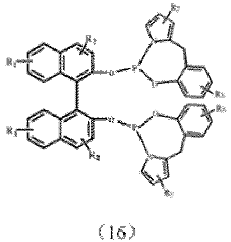 | 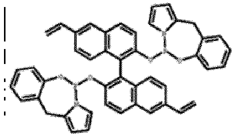 | 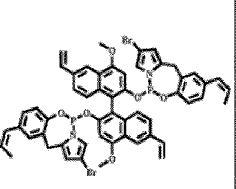 | 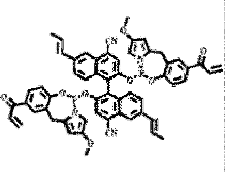 | 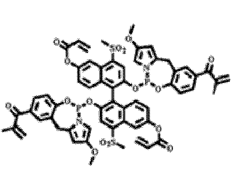 | 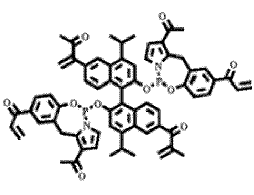 |
| 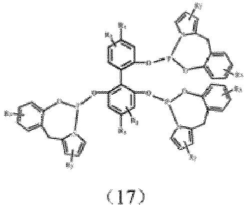 | 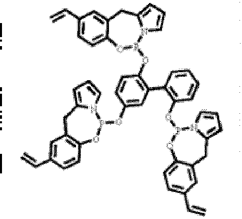 | 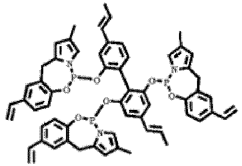 | 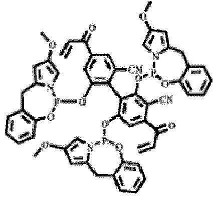 | 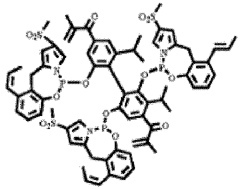 | 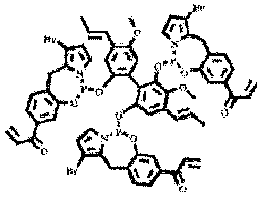 |
| 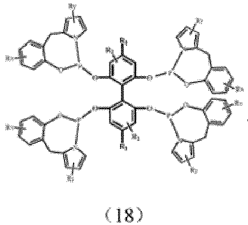 | 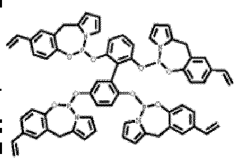 | 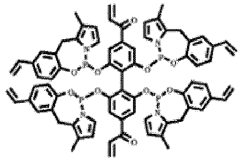 | 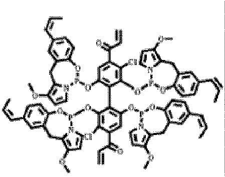 | 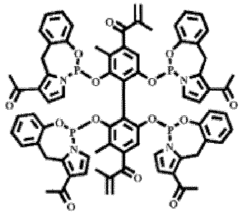 | 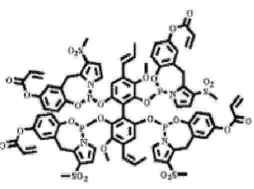 |

The porous polymer containing a phosphorous ligand according to the present disclosure is a polymer formed by at least one of the phosphorous ligand monomers described above, wherein the ligand phosphorus is enriched on the surface of the pore channel, which is conducive to the form a specific spatial structure, increases the coordination effect of phosphorus ligands. The contribution to the catalyst is to increase the reactivity and improve the selectivity of the reaction. Combining said porous polymer with its secondary pore channel structure with specific pore diameter distribution, the overall effect that may be obtained is that the catalyst has high reactivity, high selectivity, and high linearity of the product.

The present disclosure further provides a method for preparing porous polymer containing a phosphorous ligand, wherein at least one of the phosphorous ligands is self-polymerized or copolymerized in the presence of a radical initiator.

In a preferred case, the method specifically comprises: subjecting at least one of the phosphorous ligands to prepolymerization in the presence of a first organic solvent to obtain a prepolymer, wherein the difference in solubility parameter of at least one of the phosphorous ligands and the first organic solvent is 1.0 to 2.5[MPa]^{1/2}; adding a second organic solvent to the resulting prepolymer such that the difference in solubility parameter of a mixed solvent of the first and second organic solvents and the prepolymer is less than 0.5 [MPa]^{1/2}, and swelling and curing the prepolymer.

For the method for preparing porous polymer containing a phosphorous ligand according to the present disclosure, in a preferred case, the prepolymerization temperature is 50 to 80 °C, and the prepolymerization time is 2 to 10 hours; the swelling and curing temperature is 85 to 110 °C, and the time is 2 to 10 hours.

For the method for preparing porous polymer containing a phosphorous ligand according to the present disclosure, in a preferred case, any one of the phosphorous ligand is self-polymerized.

For the method for preparing porous polymer containing a phosphorous ligand according to the present disclosure, in a preferred case, any two of the phosphorous ligands are copolymerized, and the molar ratio between the two phosphorous ligands is 0.01 to 3:1, and preferably 0.05 to 2.5:1.

For the method for preparing porous polymer containing a phosphorous ligand according to the present disclosure, in a preferred case, any three or more of the phosphorous ligands are copolymerized.

In the above-mentioned method for preparing the porous polymer containing a phosphorus ligand, in the prepolymerization of the first stage, selecting an organic solvent with a large difference in the solubility parameter of the porous polymer may allow the polymer form a smaller pore diameter (< 10 nm, preferably 2 to 6 nm), which is advantageous to increasing the pore volume and specific surface area, and is advantageous to increasing the linearity of the product during the reaction; in the polymerization of the second stage, selecting an organic solvent with a small difference in the solubility parameter of the prepolymer may allow the low cross-linking moiety of the prepolymer fully swell and expand the pore diameter of the polymer (> 15 nm, preferably greater than 20 nm), so as to be conducive to the transport and internal diffusion of the reactants and products, increase the reaction speed, and increase the activity of the catalyst.

For the method for preparing porous polymer containing a phosphorous ligand according to the present disclosure, wherein the radical initiator is selected from at least one of the group consisting of 2,2'-azobisisobutyronitrile and 2,2'-azobis(2-methylpropionitrile).

For the method for preparing porous polymer containing a phosphorous ligand according to the present disclosure, wherein the first organic solvent and the second organic solvent are the same or different, and either of them is one or more selected from the group consisting of n-pentane, n-hexane, n-heptane, dodecane, cyclohexane, isobutyl acetate, benzonitrile, methyl isobutyl ketone, n-butyl acetate, cyclopentane, 3-pentanone, p-xylene, toluene, methylpropyl ketone, tetrahydrofuran, ethyl acetate, benzene, trichloromethane, 1,1,2-trichloroethane, methyl acetate, 1,2-dichloroethane, acetone, cyclohexanone, 1,4-dioxane, cyclopentanone, propionitrile, ethanol, dimethyl sulfoxide, methanol, and water.

The present disclosure further provides a porous polymer-nickel catalyst, comprising the porous polymer containing a phosphorous ligand according to the present disclosure and a zero-valent nickel, and the content of the zero-valent nickel is 0.1 to 2 mmol/g with respect to an amount of the catalyst.

The present disclosure further provides a method for preparing porous polymer-nickel catalyst, wherein the method comprises: under an inert gas atmosphere, the porous polymer containing a phosphorous ligand according to the present disclosure is mixed with an active nickel species in an organic solvent at 20 to 100° C for 6 to 24 hours. The active nickel species is cyclooctadiene nickel.

For the method for preparing the porous polymer-nickel catalyst according to the present disclosure, wherein the molar ratio of the mole number of phosphorus to the mole number of cyclooctadiene nickel in the porous polymer is 2 to 20: 1, preferably 2 to 10:1.

The present disclosure further provides a method for preparing adiponitrile, and the method comprises: sequentially subjecting to a primary hydrocyanation reaction of butadiene, an isomerization reaction of branched mononitriles, and a secondary hydrocyanation reaction of linear mononitriles in the presence of the porous polymer-nickel catalyst according to the present disclosure.

For method for preparing adiponitrile, wherein the method comprises the following steps of:
(1) primary hydrocyanation reaction
   subjecting butadiene and hydrocyanic acid to a primary hydrocyanation reaction in the presence of the catalyst, wherein the molar ratio of the butadiene to the hydrocyanic acid is 1.0 to 1.5, and the ratio of a mole number of hydrocyanic acid to a mole number of the catalyst in terms of zero-valent nickel is 100 to 1:1, preferably 70 to 10:1, and a reaction temperature is 60 to 140 °C, and a reaction pressure is 0.3 to 5.0 MPa;
(2) isomerization reaction of branched mononitriles
   subjecting a mixture of branched mononitrile separated from a product obtained in step 1 to an isomerization reaction in the presence of the catalyst, wherein the ratio of the mole number of the mixture of branched mononitriles to the mole number of the catalyst in terms of zero-valent nickel is 300 to 20:1, preferably 200 to 50:1, and the reaction temperature is 80 to 170 °C, and the reaction pressure is 0.3 to 5.0 MPa;
(3) secondary hydrocyanation reaction
   subjecting a mixture of linear mononitriles separated from the products obtained in steps (1) and (2) and hydrocyanic acid to a secondary hydrocyanation reaction in the presence of the catalyst, wherein the molar ratio of the mixture of linear mononitriles to the hydrocyanic acid is 1.0 to 1.5, the ratio of the mole number of the hydrocyanic acid to the mole number of the catalyst in terms of zero-valent nickel is 1000 to 20:1, preferably 500 to 20:1, and the reaction temperature is 30 to 120 °C, and the reaction pressure is 0.3 to 5.0 MPa.

In a preferred case, the secondary hydrocyanation reaction is performed in the presence of a promoter, and the ratio of the mole number of the promoter to the mole number of the catalyst in terms of zero-valent nickel is 0.05 to 2.5:1;
the promoter is a Lewis acid; the Lewis acid is one or more salts selected from Groups Ib, IIb, IIIa, IIIb, IVa, IVb, Va, Vb, VIb, VIIb and VIII of the Periodic Table of the Elements; and the salt is one or more selected from a halide, a sulfate, a sulfonate, a haloalkylsulfonate, a perhaloalkylsulfonate, a haloalkylacetate, a perhaloalkylacetate, a carboxylate, and a phosphate;
preferably, the Lewis acid is one or more selected from zinc chloride, zinc bromide, zinc iodide, manganese chloride, manganese bromide, cadmium chloride, cadmium bromide, stannous chloride, stannous bromide, stannous sulfate, stannous tartrate, indium trifluoromethanesulfonate, indium trifluoroacetate, zinc trifluoroacetate, chlorides or bromides of rare earth elements such as lanthanum, cerium, praseodymium, neodymium, samarium, europium, gadolinium, terbium, dysprosium, hafnium, erbium, thallium, ytterbium and lutetium, cobalt chloride, ferrous chloride, and yttrium chloride;
more preferably, the Lewis acid is one or more of zinc chloride and ferric chloride.

According to one embodiment of the present disclosure, the method for preparing adiponitrile further comprises recycling the porous polymer-nickel catalyst, and the recycling comprises the following method (a) or (b):
(a) discharging at least part of the porous polymer-nickel catalyst together with a reaction product, filtering and separating, washing, and recycling in the next batch of reactions; or
(b) discharging at least part of the porous polymer-nickel catalyst without the reaction product, and recycling the catalyst still remain the reaction system.

### Examples

The following illustrates the present disclosure in further detail by the way of specific embodiments, however, the present disclosure is not limited to the embodiments described below.

### Example 1

### Preparation of porous polymer A

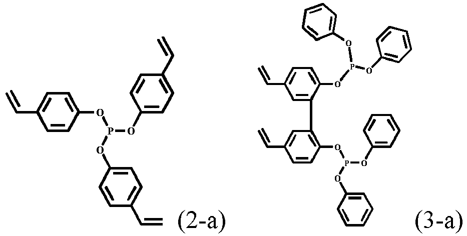

Monomer 2-a (23.3 g, 60 mmol) and monomer 3-a (40.2 g, 60 mmol) were dissolved in 800 mL of tetrahydrofuran (the difference in solubility parameter of the solvent and the mixed monomer was 2.35 MPa^{1/2}), 1.0 g of azobisisobutyronitrile (AIBN) was added, and the mixture was stirred at 50 °C to carry out prepolymerization for 4 hours to obtain a prepolymer. Then, 133 mL of n-heptane was added thereto (such that the difference in solubility parameter of the mixed solvent and the prepolymer was 0.3 MPa^{1/2}), the temperature was raised to 85 °C, and the reaction was continued for 4 hours, so that the prepolymer was swelled and fully cured. After the reaction was completed, the temperature was lowered to room temperature, and the resultant was filtered, washed with 3-pentenenitrile, and vacuum dried to obtain porous polymer A (58.4 g).

The specific surface area and pore volume of the obtained porous polymer A were measured using the BET test method. The pore diameter distribution (i.e., the ratio of the pore volume of pores with pore diameter < 10 nm to the pore volume of pores with pore diameter > 15 nm, respectively) of porous polymer A was determined by using a Micromeritics ASAP 2020 automatic physisorption analyzer with the Nonlocal density function theory (NLDFT) model. After sample digestion, the P content was determined by ammonium molybdate spectrophotometry. The specific results were shown in Table 2-1.

### (2) Preparation of porous polymer-nickel catalyst A

Under nitrogen atmosphere, porous polymer A (52.9 g, the amount of P was 150 mmol) and bis-(1,5-cyclooctadiene)nickel (8.3 g, 30 mmol) were added to 1500 mL of 3-pentenenitrile. Next, the mixture was continued to stir at room temperature for 12 hours. After the stirring was completed, the insoluble substance in the mixed solution was filtered by cake filtration, washed, and dried under vacuum to obtain porous polymer-nickel catalyst A (54.7 g). The content of Ni in catalyst A was determined by inductively coupled plasma optical emission spectrometer (ICP-OES), and the specific results were shown in Table 2-1.

### (3) Preparation of adiponitrile

### (i) Hydrocyanation reaction of butadiene

Into the reactor charged with porous polymer-nickel catalyst A (the content of Ni was 10 mmol), 20.16 g (0.40 mol) of butadiene (BD) was added, and 8.91 g (0.33 mol) of HCN was added dropwise within 0.5 hours. The mixture was reacted for 1 hour under the conditions of the reaction temperature of 75 °C and the reaction pressure of 1.2 MPa. After the reaction was completed, the product was discharged from the bottom of the reactor. After separating the catalyst by filtration, the sample was taken, and the distribution of the product was analyzed by GC to determine the conversion of starting HCN into 3-pentenenitrile (3PN) and 2-methyl-3-butenenitrile (2M3BN) and the ratio of 3-pentenenitrile (3PN) to 2-methyl-3-butenenitrile (2M3BN) (3PN/2M3BN). The results were shown in Table 2-1.

### (ii) Isomerization of 2-methyl-3-butenenitrile (2M3BN):

2M3BN was obtained by separating the reaction product of step (i), 64.90 g (0.80 mol) of 2M3BN was added into a reactor charged with porous polymer-nickel catalyst A (the content of Ni was 10 mmol). The mixture was reacted at the reaction temperature of 150° C and the reaction pressure of 0.6MPa for 2.5 hours. After the reaction was completed, the product was discharged from the bottom of the reactor. After separating the catalyst by filtration, the sample was taken, and the ratio of the product 3PN to 2M3BN was analyzed by GC (with valeronitrile as the internal standard). The results were shown in Table 2-1.

### (iii) Secondary hydrocyanation reaction of 3-pentenenitrile (3PN):

The product 3PN obtained by separation in step (i) and step (ii) was collected, and 81.12 g (1.0 mol) of 3PN and the promoter anhydrous zinc chloride 1.02 g (7.5 mmol) were added into the reactor charged with porous polymer-nickel catalyst A (the content of Ni was 5 mmol), 22.41 g (0.83 mol) of HCN was added dropwise within 5 hours, and the mixture was reacted for 10.0 hours under the conditions of the reaction temperature of 65 °C and the reaction pressure of 0.3 MPa. After the reaction was completed, the product was discharged from the bottom of the reactor. After separating the catalyst by filtration, the sample was taken, and the conversion to the product adiponitrile based on HCN, the selectivity to linear adiponitrile, and the percentage of ADN in the reaction mixture were determined by analyzing the product distribution with GC. The results were shown in Table 2-1.

### Example 2

### (1) Preparation of porous polymer B

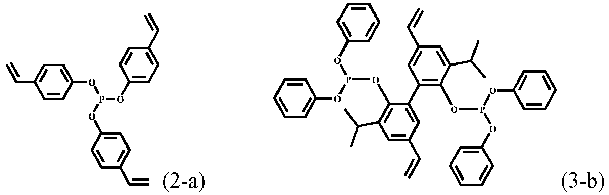

Monomer 2-a (23.3 g, 60 mmol) and monomer 3-b (45.3g, 60 mmol) were dissolved in 1000 mL of trichloromethane (the difference in solubility parameter of the solvent and the mixed monomer was 2.28 MPa^{1/2}), 1.0 g of azobisisobutyronitrile (AIBN) was added, and the mixture was stirred at 50 °C to carry out prepolymerization for 4 hours to obtain a prepolymer. Then, 200 mL of isobutyl acetate was added thereto (such that the difference in solubility parameter of the mixed solvent and the prepolymer was 0.39 MPa^{1/2}). Subsequently, porous polymer B (63.1 g) was obtained in the same manner as in Example 1.

The measurements were performed in the same manner as in Example 1, and the various performance parameters of porous polymer B were shown in Table 2-1.

### (2) Preparation of porous polymer-nickel catalyst B

Under nitrogen atmosphere, porous polymer B (57.1 g, the amount of P was 150 mmol) and bis-(1,5-cyclooctadiene)nickel (8.3 g, 30 mmol) were added to 1500 mL of 3-pentenenitrile. Subsequently, catalyst B (58.9 g) was obtained in the same manner as in Example 1, and the content of Ni in catalyst B determined in the same manner as in Example 1 was shown in Table 2-1.

### (3) Preparation of adiponitrile

Preparation and analysis were carried out in the same manner as in Example 1 except that catalyst B was used. The analysis was carried out in the same manner as in Example 1, and the results were shown in Table 2-1.

### Example 3

### (1) Preparation of porous polymer C

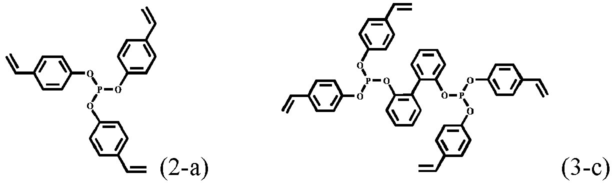

Monomer 2-a (23.3 g, 60 mmol) and monomer 3-c (43.3 g, 60 mmol) were dissolved in 1000 mL of benzene (the difference in solubility parameter of the solvent and the mixed monomer was 2.46 MPa^{1/2}), 1.0 g of azobisisobutyronitrile (AIBN) was added, and the mixture was stirred at 50 °C to carry out prepolymerization for 4 hours to obtain a prepolymer. Then, 1000 mL of dodecane was added thereto (such that the difference in solubility parameter of the mixed solvent and the prepolymer was 0.05 MPa^{1/2}), and porous polymer C (58.6 g) was obtained in the same manner as in Example 1.

The measurements were performed in the same manner as in Example 1, and the various performance parameters of porous polymer C were shown in Table 2-1.

### (2) Preparation of porous polymer-nickel catalyst C

Under nitrogen atmosphere, porous polymer C (22.2 g, the amount of P was 60 mmol) and bis-(1,5-cyclooctadiene)nickel (8.3 g, 30 mmol) were added to 1500 mL of 3-pentenenitrile. Subsequently, catalyst C (24.0 g) was obtained in the same manner as in Example 1, and the content of Ni in catalyst C determined in the same manner as in Example 1 was shown in Table 2-1.

### (3) Preparation of adiponitrile

Preparation and analysis were carried out in the same manner as in Example 1 except that catalyst C was used. The analysis was carried out in the same manner as in Example 1, and the results were shown in Table 2-1.

### Example 4

### (1) Preparation of porous polymer D

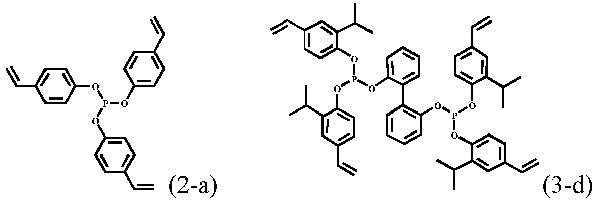

Monomer 2-a (23.3 g, 60 mmol) and monomer 3-d (53.4 g, 60 mmol) were dissolved in 1200 mL of trichloroethane (the difference in solubility parameter of the solvent and the mixed monomer was 2.25 MPa^{1/2}), 1.0 g of azobisisobutyronitrile (AIBN) was continuously added, and the mixture was stirred at 50 °C to carry out prepolymerization for 4 hours to obtain a prepolymer. Then, 400 mL of benzonitrile was added thereto (such that the difference in solubility parameter of the mixed solvent and the prepolymer was 0.48 MPa^{1/2}), and porous polymer D (70.6g) was obtained in the same manner as in Example 1.

The measurements were performed in the same manner as in Example 1, and the various performance parameters of porous polymer D were shown in Table 2-1.

### (2) Preparation of porous polymer-nickel catalyst D

Under nitrogen atmosphere, porous polymer D (63.9 g, the amount of P was 150 mmol) and bis-(1,5-cyclooctadiene)nickel (8.3 g, 30 mmol) were added to 1500 mL of 3-pentenenitrile. Subsequently, catalyst D (65.7g) was obtained in the same manner as in Example 1, and the content of Ni in catalyst D determined in the same manner as in Example 1 was shown in Table 2-1.

### (3) Preparation of adiponitrile

Preparation and analysis were carried out in the same manner as in Example 1 except that catalyst D was used. The analysis was carried out in the same manner as in Example 1, and the results were shown in Table 2-1.

### Example 5

### (1) Preparation of porous polymer E

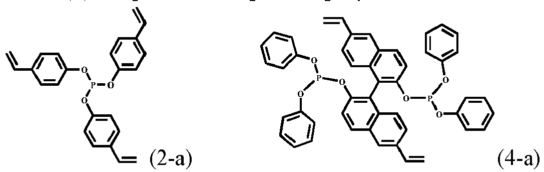

Monomer 2-a (23.3 g, 60 mmol) and monomer 4-a (46.2 g, 60 mmol) were dissolved in 800 mL of ethyl acetate (the difference in solubility parameter of the solvent and the mixed monomer was1.77 MPa^{1/2}), 1.0 g of azobisisobutyronitrile (AIBN) was added, and the mixture was stirred at 50 °C to carry out prepolymerization for 4 hours to obtain a prepolymer. Then, 200 mL of methyl isobutyl ketone was added thereto (such that the difference in solubility parameter of the mixed solvent and the prepolymer was 0.04 MPa^{1/2}), and porous polymer E (62.0 g) was obtained in the same manner as in Example 1.

The measurements were performed in the same manner as in Example 1, and the various performance parameters of porous polymer E were shown in Table 2-1.

### (2) Preparation of porous polymer-nickel catalyst E

Under nitrogen atmosphere, porous polymer E (57.9 g, the amount of P was 150 mmol) and bis-(1,5-cyclooctadiene)nickel (8.3 g, 30 mmol) were added to 1500 mL of 3-pentenenitrile. Subsequently, catalyst E (59.7g) was obtained in the same manner as in Example 1. The content of Ni in catalyst E determined in the same manner as in Example 1 was shown in Table 2-1.

### (3) Preparation of adiponitrile

Preparation and analysis were carried out in the same manner as in Example 1 except that catalyst E was used. The analysis was carried out in the same manner as in Example 1, and the results were shown in Table 2-1.

### Example 6

### (1) Preparation of porous polymer F

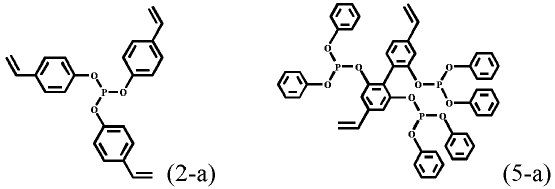

Monomer 2-a (23.3 g, 60 mmol) and monomer 5-a (54.1 g, 60 mmol) were dissolved in 1200 mL of 3-pentanone (the difference in solubility parameter of the solvent and the mixed monomer was 2.2 MPa^{1/2}), 1.0 g of azobisisobutyronitrile (AIBN) was added, and the mixture was stirred at 50 °C to carry out prepolymerization for 4 hours to obtain a prepolymer. Then, 300 mL of n-butyl acetate (such that the difference in solubility parameter of the mixed solvent and the prepolymer was 0.23 MPa^{1/2}), and porous polymer F (68.1 g) was obtained in the same manner as in Example 1.

The measurements were performed in the same manner as in Example 1, and the various performance parameters of porous polymer F were shown in Table 2-1.

### (2) Preparation of porous polymer-nickel catalyst F

Under nitrogen atmosphere, porous polymer F (58.1 g, the amount of P was 180 mmol) and bis-(1,5-cyclooctadiene)nickel (8.3 g, 30 mmol) were added to 1500 mL of 3-pentenenitrile. Subsequently, catalyst F (59.9 g) was obtained in the same manner as in Example 1, and the content of Ni in catalyst F determined in the same manner as in Example 1 was shown in Table 2-1.

### (3) Preparation of adiponitrile

Preparation and analysis were carried out in the same manner as in Example 1 except that catalyst F was used. The analysis was carried out in the same manner as in Example 1, and the results were shown in Table 2-1.

### Example 7

### (1) Preparation of porous polymer G

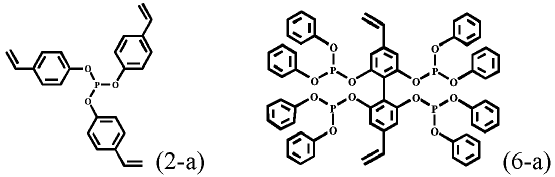

Monomer 2-a (23.3 g, 60 mmol) and monomer 6-a (68.1 g, 60 mmol) were dissolved in 1500 mL of p-xylene (the difference in solubility parameter of the solvent and the mixed monomer was 2.00 MPa^{1/2}), 1.0 g of azobisisobutyronitrile (AIBN) was added, and the mixture was stirred at 50 °C to carry out prepolymerization for 4 hours to obtain a prepolymer. Then, 500 mL of isobutyl acetate was added thereto (such that the difference in solubility parameter of the mixed solvent and the prepolymer was 0.03 MPa^{1/2}), and porous polymer G (80.4 g) was obtained in the same manner as in Example 1.

The measurements were performed in the same manner as in Example 1, and the various performance parameters of porous polymer G were shown in Table 2-1.

### (2) Preparation of porous polymer-nickel catalyst G

Under nitrogen atmosphere, porous polymer G (45.7 g, the amount of P was 150 mmol) and bis-(1,5-cyclooctadiene)nickel (8.3 g, 30 mmol) were added to 1500 mL of 3-pentenenitrile. Subsequently, catalyst G (47.5 g) was obtained in the same manner as in Example 1, and the content of Ni in catalyst G determined in the same manner as in Example 1 was shown in Table 2-1.

### (3) Preparation of adiponitrile

Preparation and analysis were carried out in the same manner as in Example 1 except that catalyst G was used. The analysis was carried out in the same manner as in Example 1, and the results were shown in Table 2-1.

### Example 8

### (1) Preparation of porous polymer H

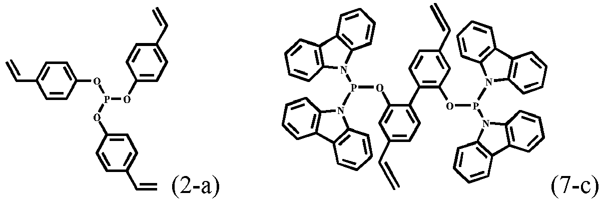

Monomer 2-a (23.3 g, 60 mmol) and monomer 7-c (57.7 g, 60 mmol) were dissolved in 1200 mL of 1,2-dichloroethane (the difference in solubility parameter of the solvent and the mixed monomer was 2.15 MPa^{1/2}), 1.0 g of azobisisobutyronitrile (AIBN) was added, and the mixture was stirred at 50 °C to carry out prepolymerization for 4 hours to obtain a prepolymer. Then, 300 mL of propionitrile was added thereto (such that the difference in solubility parameter of the mixed solvent and the prepolymer was 0.22 MPa^{1/2}), and porous polymer H (73.7 g) was obtained in the same manner as in Example 1.

The measurements were performed in the same manner as in Example 1, and the various performance parameters of porous polymer H were shown in Table 2-1.

### (2) Preparation of porous polymer-nickel catalyst H

Under nitrogen atmosphere, porous polymer H (67.5 g, the amount of P was 150 mmol) and bis-(1,5-cyclooctadiene)nickel (8.3 g, 30 mmol) were added to 1500 mL of 3-pentenenitrile. Subsequently, catalyst H (69.3 g) was obtained in the same manner as in Example 1, and the content of Ni in catalyst H determined in the same manner as in Example 1 was shown in Table 2-1.

### (3) Preparation of adiponitrile

Preparation and analysis were carried out in the same manner as in Example 1 except that catalyst H was used. The analysis was carried out in the same manner as in Example 1, and the results were shown in Table 2-1.

### Example 9

### (1) Preparation of porous polymer I

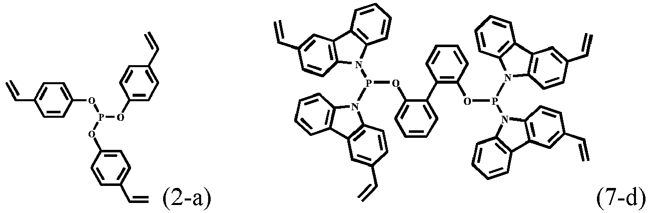

Monomer 2-a (23.3 g, 60 mmol) and monomer 7-d (60.9 g, 60 mmol) were dissolved in 1500 mL 1,4-dioxane (the difference in solubility parameter of the solvent and the mixed monomer was 2.23 MPa^{1/2}), 1.0 g of azobisisobutyronitrile (AIBN) was added, and the mixture was stirred at 50 °C to carry out prepolymerization for 4 hours to obtain a prepolymer. Then, 300 mL of cyclohexanone was added thereto (such that the difference in solubility parameter of the mixed solvent and the prepolymer was 0.48 MPa^{1/2}), and porous polymer I (73.2 g) was obtained in the same manner as in Example 1.

The measurements were performed in the same manner as in Example 1, and the various performance parameters of porous polymer I were shown in Table 2-1.

### (2) Preparation of porous polymer-nickel catalyst I

Under nitrogen atmosphere, porous polymer I (70.1 g, the amount of P was 150 mmol) and bis-(1,5-cyclooctadiene)nickel (8.3 g, 30 mmol) were added to 1500 mL of 3-pentenenitrile. Subsequently, catalyst I sample (71.9 g) was obtained in the same manner as in Example 1, and the content of Ni in catalyst I determined in the same manner as in Example 1 was shown in Table 2-1.

### (3) Preparation of adiponitrile

Preparation and analysis were carried out in the same manner as in Example 1 except that catalyst I was used. The analysis was carried out in the same manner as in Example 1, and

### Example 10

### (1) Preparation of porous polymer J

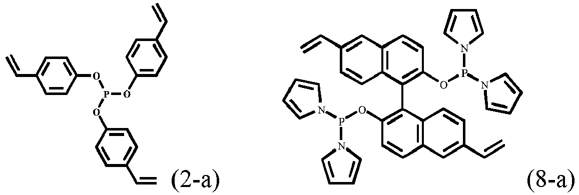

Monomer 2-a (23.3 g, 60 mmol) and monomer 8-a (39.7 g, 60 mmol) were dissolved in 800 mL of trichloromethane (the difference in solubility parameter of the solvent and the mixed monomer was 1.91 MPa^{1/2}), 1.0 g of azobisisobutyronitrile (AIBN) was added, and the mixture was stirred at 50 °C to carry out prepolymerization for 4 hours to obtain a prepolymer. Then, 200 mL of methyl propyl ketone was added thereto (such that the difference in solubility parameter of the mixed solvent and the prepolymer was 0.36 MPa^{1/2}), and porous polymer J (58.0 g) was obtained in the same manner as in Example 1.

The measurements were performed in the same manner as in Example 1, and the various performance parameters of porous polymer J were shown in Table 2-1.

### (2) Preparation of porous polymer-nickel catalyst J

Under nitrogen atmosphere, porous polymer J (52.5 g, the amount of P was 150 mmol) and bis-(1,5-cyclooctadiene)nickel (8.3 g, 30 mmol) were added to 1500 mL of 3-pentenenitrile. Subsequently, catalyst J sample (54.3 g) was obtained in the same manner as in Example 1, and the content of Ni in catalyst J determined in the same manner as in Example 1 was shown in Table 2-1.

### (3) Preparation of adiponitrile

Preparation and analysis were carried out in the same manner as in Example 1 except that catalyst J was used. The analysis was carried out in the same manner as in Example 1, and the results were shown in Table 2-1.

### Example 11

### (1) Preparation of porous polymer K

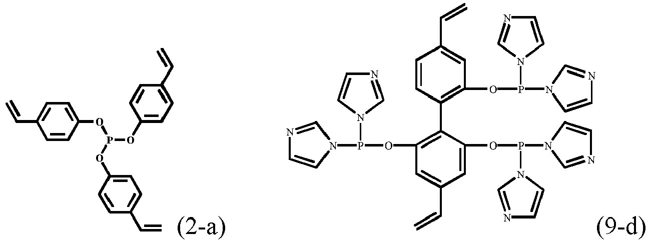

Monomer 2-a (23.3 g, 60 mmol) and monomer 9-d (44.8 g, 60 mmol) were dissolved in 800 mL of p-xylene (the difference in solubility parameter of the solvent and the mixed monomer was 2.00 MPa^{1/2}), 1.0 g of azobisisobutyronitrile (AIBN) was added, and the mixture was stirred at 50 °C to carry out prepolymerization for 4 hours to obtain a prepolymer. Then, 800 mL of isobutyl acetate was added thereto (such that the difference in solubility parameter of the mixed solvent and the prepolymer was 0.30 MPa^{1/2}), porous polymer K (61.3 g) was obtained in the same manner as in Example 1.

The measurements were performed in the same manner as in Example 1, and the various performance parameters of porous polymer K were shown in Table 2-1.

### (2) Preparation of porous polymer-nickel catalyst K

Under nitrogen atmosphere, porous polymer K (42.5 g, the amount of P was 150 mmol) and bis-(1,5-cyclooctadiene)nickel (8.3 g, 30 mmol) were added to 1500 mL of 3-pentenenitrile. Subsequently, catalyst K (44.3 g) was obtained in the same manner as in Example 1, and the content of Ni in catalyst K determined in the same manner as in Example 1 was shown in Table 2-1.

### (3) Preparation of adiponitrile

Preparation and analysis were carried out in the same manner as in Example 1 except that catalyst K was used. The analysis was carried out in the same manner as in Example 1, and the results were shown in Table 2-1.

### Example 12

### (1) Preparation of porous polymer L

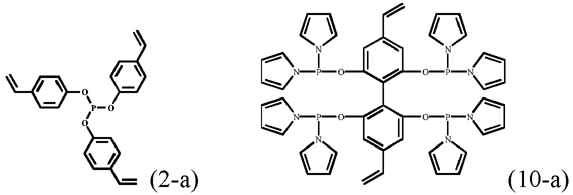

Monomer 2-a (23.3 g, 60 mmol) and monomer 10-a (55.1 g, 60 mmol) were dissolved in 1200 mL of ethyl acetate (the difference in solubility parameter of the solvent and the mixed monomer was1.96 MPa^{1/2}), 1.0 g of azobisisobutyronitrile (AIBN) was added, and the mixture was stirred at 50 °C to carry out prepolymerization for 4 hours to obtain a prepolymer. Then, 300 mL of methyl isobutyl ketone was added thereto (such that the difference in solubility parameter of the mixed solvent and the prepolymer was 0.2M Pa^{1/2}), porous polymer L (71.5 g) was obtained in the same manner as in Example 1.

The measurements were performed in the same manner as in Example 1, and the various performance parameters of porous polymer L were shown in Table 2-1.

### (2) Preparation of porous polymer-nickel catalyst L

Under nitrogen atmosphere, porous polymer L (70.5 g, the amount of P was 270 mmol) and bis-(1,5-cyclooctadiene)nickel (7.5 g, 27 mmol) were added to 1500 mL of 3-pentenenitrile. Subsequently, catalyst L (72.3 g) was obtained in the same manner as in Example 1, and the content of Ni in catalyst L determined in the same manner as in Example 1 was shown in Table 2-1.

### (3) Preparation of adiponitrile

Preparation and analysis were carried out in the same manner as in Example 1 except that catalyst L was used. The analysis was carried out in the same manner as in Example 1, and the results were shown in Table 2-1.

### Example 13

### (1) Preparation of porous polymer M

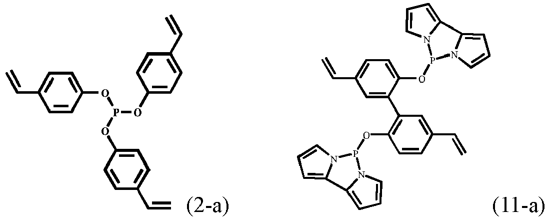

Monomer 2-a (23.3 g, 60 mmol) and monomer 11-a (33.5 g, 60 mmol) were dissolved in 800 mL of methyl propyl ketone (the difference in solubility parameter of the solvent and the mixed monomer was 1.34 MPa^{1/2}), 1.0 g of azobisisobutyronitrile (AIBN) was added, and the mixture was stirred at 50 °C to carry out prepolymerization for 4 hours to obtain a prepolymer. Then, 800 mL of toluene was added thereto (such that the difference in solubility parameter of the mixed solvent and the prepolymer was 0.14 MPa^{1/2}), and porous polymer M (51.7 g) was obtained in the same manner as in Example 1.

The measurements were performed in the same manner as in Example 1, and the various performance parameters of porous polymer M were shown in Table 2-1.

### (2) Preparation of porous polymer-nickel catalyst M

Under nitrogen atmosphere, porous polymer M (47.3 g, the amount of P was 150 mmol) and bis-(1,5-cyclooctadiene)nickel (8.3 g, 30 mmol) were added to 1500 mL of 3-pentenenitrile. Subsequently, catalyst M (49.1 g) was obtained in the same manner as in Example 1, and the content of Ni in catalyst M determined in the same manner as in Example 1 was shown in Table 2-1.

### (3) Preparation of adiponitrile

Preparation and analysis were carried out in the same manner as in Example 1 except that catalyst M was used. The analysis was carried out in the same manner as in Example 1, and the results were shown in Table 2-1.

### Example 14

### (1) Preparation of porous polymer N

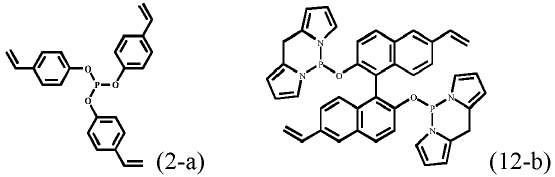

Monomer 2-a (23.3 g, 60 mmol) and monomer 12-b(41.2 g, 60 mmol) were dissolved in 800 mL of trichloromethane (the difference in solubility parameter of the solvent and the mixed monomer was 1.74 MPa^{1/2}), 1.0 g of azobisisobutyronitrile (AIBN) was added, and the mixture was stirred at 50 °C to carry out prepolymerization for 4 hours to obtain a prepolymer. Then, 200 mL of methyl propyl ketone was added thereto (such that the difference in solubility parameter of the mixed solvent and the prepolymer was 0.03 MPa^{1/2}), and porous polymer N (56.7 g) was obtained in the same manner as in Example 1.

The measurements were performed in the same manner as in Example 1, and the various performance parameters of porous polymer N were shown in Table 2-1.

### (2) Preparation of porous polymer-nickel catalyst N

Under nitrogen atmosphere, porous polymer N (53.7 g, the amount of P was 150 mmol) and bis-(1,5-cyclooctadiene)nickel (8.3 g, 30 mmol) were added to 1500 mL of 3-pentenenitrile. Subsequently, catalyst N (55.5 g) was obtained in the same manner as in Example 1, and the content of Ni in catalyst N determined in the same manner as in Example 1 was shown in Table 2-1.

### (3) Preparation of adiponitrile

Preparation and analysis were carried out in the same manner as in Example 1 except that catalyst N was used. The analysis was carried out in the same manner as in Example 1, and the results were shown in Table 2-1.

### Example 15

### (1) Preparation of porous polymer O

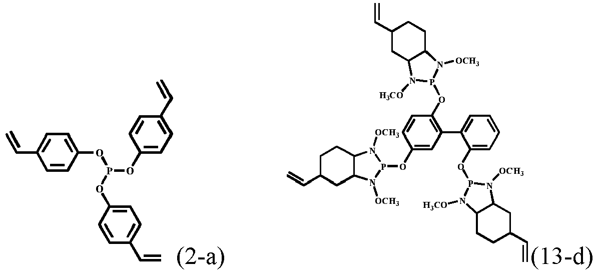

Monomer 2-a (23.3 g, 60 mmol) and monomer 13-d (53.2 g, 60 mmol) were dissolved in 1000 mL of trichloromethane (the difference in solubility parameter of the solvent and the mixed monomer was 1.8 MPa^{1/2}), 1.0 g of azobisisobutyronitrile (AIBN) was added, and the mixture was stirred at 50 °C to carry out prepolymerization for 4 hours to obtain a prepolymer. Then, 250 mL of methyl propyl ketone was added thereto (such that the difference in solubility parameter of the mixed solvent and the prepolymer was 0.06 MPa^{1/2}), and porous polymer O (66.5 g) was obtained in the same manner as in Example 1.

The measurements were performed in the same manner as in Example 1, and the various performance parameters of porous polymer O were shown in Table 2-1.

### (2) Preparation of porous polymer-nickel catalyst O

Under nitrogen atmosphere, porous polymer O (47.8 g, the amount of P was 150 mmol) and bis-(1,5-cyclooctadiene)nickel (8.3 g, 30 mmol) were added to 1500 mL of 3-pentenenitrile. Subsequently, catalyst O sample (49.6 g) was obtained in the same manner as in Example 1, and the content of Ni in catalyst O determined in the same manner as in Example 1 was shown in Table 2-1.

### (3) Preparation of adiponitrile

Preparation and analysis were carried out in the same manner as in Example 1 except that catalyst O was used. The analysis was carried out in the same manner as in Example 1, and the results were shown in Table 2-1.

### Example 16

### (1) Preparation of porous polymer P

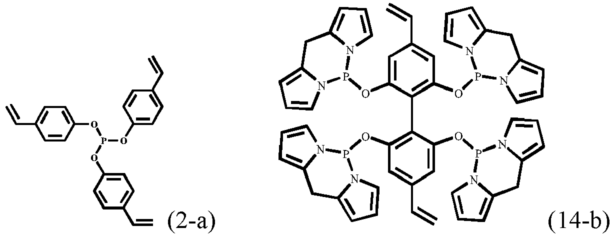

Monomer 2-a (23.3 g, 60 mmol) and monomer 14-b (58.0 g, 60 mmol) were dissolved in 1000 mL of trichloromethane (the difference in solubility parameter of the solvent and the mixed monomer was 1.74 MPa^{1/2}), 1.0 g of azobisisobutyronitrile (AIBN) was added, and the mixture was stirred at 50 °C to carry out prepolymerization for 4 hours to obtain a prepolymer. Then, 250 mL of methyl propyl ketone was added thereto (such that the difference in solubility parameter of the mixed solvent and the prepolymer was 0.34 MPa^{1/2}), and porous polymer P (69.9 g) was obtained in the same manner as in Example 1.

The measurements were performed in the same manner as in Example 1, and the various performance parameters of porous polymer P were shown in Table 2-1.

### (2) Preparation of porous polymer-nickel catalyst P

Under nitrogen atmosphere, porous polymer P (40.6 g, the amount of P was 150 mmol) and bis-(1,5-cyclooctadiene)nickel (8.3 g, 30 mmol) were added to 1500 mL of 3-pentenenitrile. Subsequently, catalyst P (42.4 g) was obtained in the same manner as in Example 1, and the content of Ni in catalyst P determined in the same manner as in Example 1 was shown in Table 2-1.

### (3) Preparation of adiponitrile

Preparation and analysis were carried out in the same manner as in Example 1 except that catalyst P was used. The analysis was carried out in the same manner as in Example 1, and the results were shown in Table 2-1.

### Example 17

### (1) Preparation of porous polymer Q

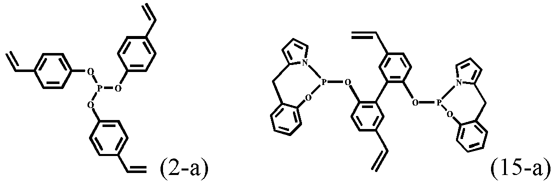

Monomer 2-a (23.3 g, 60 mmol) and monomer 15-a (38.4 g, 60 mmol) were dissolved in 800 mL of toluene (the difference in solubility parameter of the solvent and the mixed monomer was 1.51 MPa^{1/2}), 1.0 g of azobisisobutyronitrile (AIBN) was added, and the mixture was stirred at 50 °C to carry out prepolymerization for 4 hours to obtain a prepolymer. Then, 800 mL of p-xylene was added thereto (such that the difference in solubility parameter of the mixed solvent and the prepolymer was 0.2 MPa^{1/2}), and porous polymer Q (55.5 g) was obtained in the same manner as in Example 1.

The measurements were performed in the same manner as in Example 1, and the various performance parameters of porous polymer Q were shown in Table 2-1.

### (2) Preparation of porous polymer-nickel catalyst Q

Under nitrogen atmosphere, porous polymer Q (51.4 g, the amount of P was 150 mmol) and bis-(1,5-cyclooctadiene)nickel (8.3 g, 30 mmol) were added to 1500 mL of 3-pentenenitrile. Subsequently, catalyst Q (53.2 g) was obtained in the same manner as in Example 1, and the content of Ni in catalyst Q determined in the same manner as in Example 1 was shown in Table 2-1.

### (3) Preparation of adiponitrile

Preparation and analysis were carried out in the same manner as in Example 1 except that catalyst Q was used. The analysis was carried out in the same manner as in Example 1, and the results were shown in Table 2-1.

### Example 18

### (1) Preparation of porous polymer R

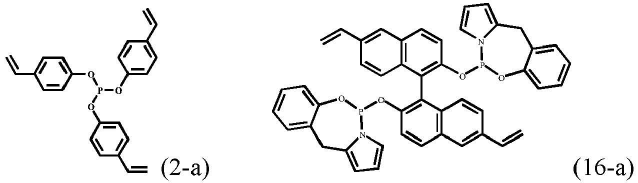

Monomer 2-a (23.3 g, 60 mmol) and monomer 16-a (44.4 g, 60 mmol) were dissolved in 800 mL of tetrahydrofuran (the difference in solubility parameter of the solvent and the mixed monomer was 1.48 MPa^{1/2}), 1.0 g of azobisisobutyronitrile (AIBN) was added, and the mixture was stirred at 50 °C to carry out prepolymerization for 4 hours to obtain a prepolymer. Then, 200 mL of n-heptane was added thereto (such that the difference in solubility parameter of the mixed solvent and the prepolymer was 0.47 MPa^{1/2}), and porous polymer R (60.2 g) was obtained in the same manner as in Example 1.

The measurements were performed in the same manner as in Example 1, and the various performance parameters of porous polymer R were shown in Table 2-1.

### (2) Preparation of porous polymer-nickel catalyst R

Under nitrogen atmosphere, porous polymer R (56.4 g, the amount of P was 150 mmol) and bis-(1,5-cyclooctadiene)nickel (8.3 g, 30 mmol) were added to 1500 mL of 3-pentenenitrile. Subsequently, catalyst R (58.2 g) was obtained in the same manner as in Example 1, and the content of Ni in catalyst R determined in the same manner as in Example 1 was shown in Table 2-1.

### (3) Preparation of adiponitrile

Preparation and analysis were carried out in the same manner as in Example 1 except that catalyst R was used. The analysis was carried out in the same manner as in Example 1, and the results were shown in Table 2-1.

### Example 19

### (1) Preparation of porous polymer S

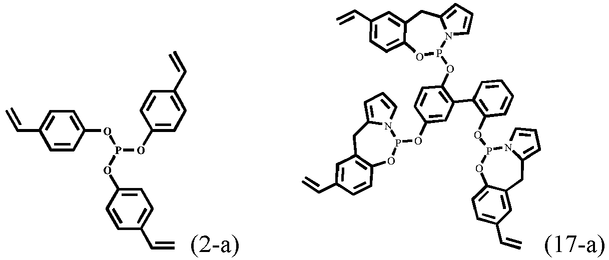

Monomer 2-a (23.3 g, 60 mmol) and monomer 17-a (53.0 g, 60 mmol) were dissolved in 1000 mL of tetrahydrofuran (the difference in solubility parameter of the solvent and the mixed monomer was 1.65 MPa^{1/2}), 1.0 g of azobisisobutyronitrile (AIBN) was added, and the mixture was stirred at 50 °C to carry out prepolymerization for 4 hours to obtain a prepolymer. Then, 250 mL of n-heptane was added thereto (such that the difference in solubility parameter of the mixed solvent and the prepolymer was 0.05 MPa^{1/2}), and porous polymer S (66.4 g) was obtained in the same manner as in Example 1.

The measurements were performed in the same manner as in Example 1, and the various performance parameters of porous polymer S were shown in Table 2-2.

### (2) Preparation of porous polymer-nickel catalyst S

Under nitrogen atmosphere, porous polymer S (47.7 g, the amount of P was 150 mmol) and bis-(1,5-cyclooctadiene)nickel (8.3 g, 30 mmol) were added to 1500 mL of 3-pentenenitrile. Subsequently, catalyst S (49.5 g) was obtained in the same manner as in Example 1, and the content of Ni in catalyst S determined in the same manner as in Example 1 was shown in Table 2-2.

### (3) Preparation of adiponitrile

Preparation and analysis were carried out in the same manner as in Example 1 except that catalyst S was used. The analysis was carried out in the same manner as in Example 1, and the results were shown in Table 2-2.

### Example 20

### (1) Preparation of porous polymer T

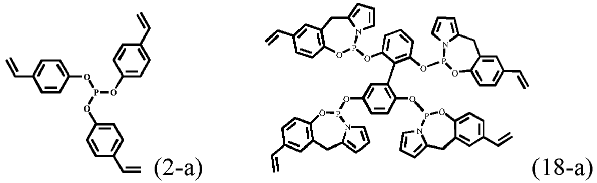

Monomer 2-a (23.3 g, 60 mmol) and monomer 18-a (67.6 g, 60 mmol) were dissolved in 1500 mL of tetrahydrofuran (the difference in solubility parameter of the solvent and the mixed monomer was 1.58 MPa^{1/2}), 1.0 g of azobisisobutyronitrile (AIBN) was added, and the mixture was stirred at 50 °C to carry out prepolymerization for 4 hours to obtain a prepolymer. Then, 300 mL of n-heptane (such that the difference in solubility parameter of the mixed solvent and the prepolymer was 0.34 MPa^{1/2}), and porous polymer T (82.6 g) was obtained in the same manner as in Example 1.

The measurements were performed in the same manner as in Example 1, and the various performance parameters of porous polymer T were shown in Table 2-2.

### (2) Preparation of porous polymer-nickel catalyst T

Under nitrogen atmosphere, porous polymer T (45.4 g, the amount of P was 150 mmol) and bis-(1,5-cyclooctadiene)nickel (8.3 g, 30 mmol) were added to 1500 mL of 3-pentenenitrile. Subsequently, catalyst T (47.2 g) was obtained in the same manner as in Example 1, and the content of Ni in catalyst T determined in the same manner as in Example 1 was shown in Table 2-2.

### (3) Preparation of adiponitrile

Preparation and analysis were carried out in the same manner as in Example 1 except that catalyst T was used. The analysis was carried out in the same manner as in Example 1, and the results were shown in Table 2-2.

### Example 21

### (1) Preparation of porous polymer A1

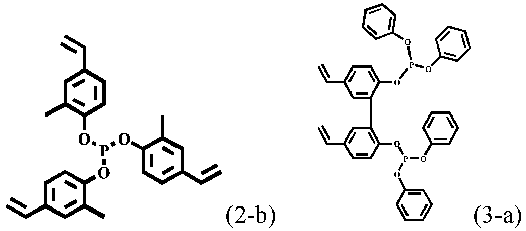

Monomer 2-b (25.8 g, 60 mmol) and monomer 3-a (40.2 g, 60 mmol) were dissolved in 800 mL of tetrahydrofuran (the difference in solubility parameter of the solvent and the mixed monomer was 1.59 MPa^{1/2}), 1.0 g of azobisisobutyronitrile (AIBN) was added, and the mixture was stirred at 50 °C to carry out prepolymerization for 4 hours to obtain a prepolymer. Then, 133 mL of n-heptane was added thereto (such that the difference in solubility parameter of the mixed solvent and the prepolymer was 0.22 MPa^{1/2}), and porous polymer A1 (58.7 g) was obtained in the same manner as in Example 1.

The measurements were performed in the same manner as in Example 1, and the various performance parameters of porous polymer A1 were shown in Table 2-2.

### (2) Preparation of porous polymer-nickel catalyst A1

Under nitrogen atmosphere, porous polymer A1 (55.0 g, the amount of P was 150 mmol) and bis-(1,5-cyclooctadiene)nickel (8.3 g, 30 mmol) were added to 1500 mL of 3-pentenenitrile. Subsequently, catalyst A1 (56.8 g) was obtained in the same manner as in Example 1, and the content of Ni in catalyst A1 determined in the same manner as in Example 1 was shown in Table 2-2.

### (3) Preparation of adiponitrile

Preparation and analysis were carried out in the same manner as in Example 1 except that catalyst A1 was used. The analysis was carried out in the same manner as in Example 1, and the results were shown in Table 2-2.

### Example 22

### (1) Preparation of porous polymer A2

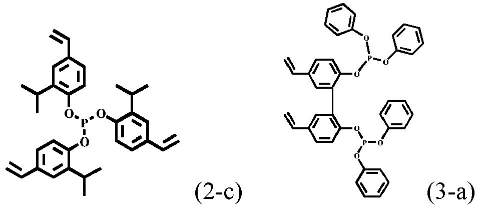

Monomer 2-c (30.9 g, 60 mmol) and monomer 3-a (40.2 g, 60 mmol) were dissolved in 800 mL of tetrahydrofuran (the difference in solubility parameter of the solvent and the mixed monomer was 1.37 MPa^{1/2}), 1.0 g of azobisisobutyronitrile (AIBN) was added, and the mixture was stirred at 50 °C to carry out prepolymerization for 4 hours to obtain a prepolymer. Then, 133 mL of n-heptane was added thereto (such that the difference in solubility parameter of the mixed solvent and the prepolymer was 0.43 MPa^{1/2}), porous polymer A2 (63.2 g) was obtained in the same manner as in Example 1.

The measurements were performed in the same manner as in Example 1, and the various performance parameters of porous polymer A2 were shown in Table 2-2.

### (2) Preparation of porous polymer-nickel catalyst A2

Under nitrogen atmosphere, porous polymer A2 (59.2 g, the amount of P was 150 mmol) and bis-(1,5-cyclooctadiene)nickel (8.3 g, 30 mmol) were added to 1500 mL of 3-pentenenitrile. Subsequently, catalyst A2 (61.0 g) was obtained in the same manner as in Example 1, and the content of Ni in catalyst A2 determined in the same manner as in Example 1 was shown in Table 2-2.

### (3) Preparation of adiponitrile

Preparation and analysis were carried out in the same manner as in Example 1 except that catalyst A2 was used. The analysis was carried out in the same manner as in Example 1, and the results were shown in Table 2-2.

### Example 23

### (1) Preparation of porous polymer A3

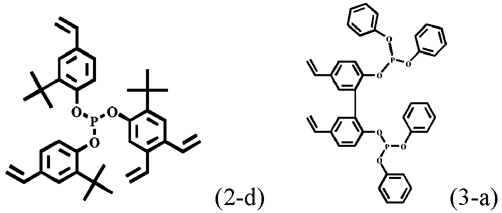

Monomer 2-d (33.4 g, 60 mmol) and monomer 3-a (40.2 g, 60 mmol) were dissolved in 800 mL of tetrahydrofuran (the difference in solubility parameter of the solvent and the mixed monomer was 1.44 MPa^{1/2}), 1.0 g of azobisisobutyronitrile (AIBN) was added, and the mixture was stirred at 50 °C to carry out prepolymerization for 4 hours to obtain a prepolymer. Then, 133 mL of n-heptane was added thereto (such that the difference in solubility parameter of the mixed solvent and the prepolymer was 0.3 MPa^{1/2}), and porous polymer A3 (67.7 g) was obtained in the same manner as in Example 1.

The measurements were performed in the same manner as in Example 1, and the various performance parameters of porous polymer A3 were shown in Table 2-2.

### (2) Preparation of porous polymer-nickel catalyst A3

Under nitrogen atmosphere, porous polymer A3 (61.3 g, the amount of P was 150 mmol) and bis-(1,5-cyclooctadiene)nickel (8.3 g, 30 mmol) were added to 1500 mL of 3-pentenenitrile. Subsequently, catalyst A3 (63.1 g) was obtained in the same manner as in Example 1, and the content of Ni in catalyst A3 determined in the same manner as in Example 1 was shown in Table 2-2.

### (3) Preparation of adiponitrile

Preparation and analysis were carried out in the same manner as in Example 1 except that catalyst A3 was used. The analysis was carried out in the same manner as in Example 1, and the results were shown in Table 2-2.

### Example 24

### (1) Preparation of porous polymer U

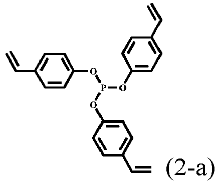

Monomer 2-a (69.9 g, 180 mmol) was dissolved in 1200 mL of tetrahydrofuran (the difference in solubility parameter of the solvent and the monomer was 2.41 MPa^{1/2}), 1.5 g of azobisisobutyronitrile (AIBN) was added, and the mixture was stirred at 50 °C to carry out prepolymerization for 4 hours to obtain a prepolymer. Then, 300 mL of n-heptane was added thereto (such that the difference in solubility parameter of the mixed solvent and the prepolymer was 0.3 MPa^{1/2}), and porous polymer U (62.3 g) was obtained in the same manner as in Example 1.

The measurements were performed in the same manner as in Example 1, and the various performance parameters of porous polymer U were shown in Table 2-2.

### (2) Preparation of porous polymer-nickel catalyst U

Under nitrogen atmosphere, porous polymer U (58.2 g, the amount of P was 150 mmol) and bis-(1,5-cyclooctadiene)nickel (8.3 g, 30 mmol) were added to 1500 mL of 3-pentenenitrile. Subsequently, catalyst U (59.9 g) was obtained in the same manner as in Example 1, and the content of Ni in catalyst U determined in the same manner as in Example 1 was shown in Table 2-2.

### (3) Preparation of adiponitrile

Preparation and analysis were carried out in the same manner as in Example 1 except that catalyst U was used. The analysis was carried out in the same manner as in Example 1, and the results were shown in Table 2-2.

### Example 25

### (1) Preparation of porous polymer V

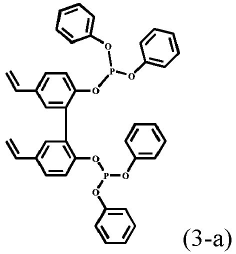

Monomer 3-a (80.4 g, 120 mmol) was dissolved in 1200 mL of tetrahydrofuran (the difference in solubility parameter of the solvent and the monomer was 2.29 MPa^{1/2}), 1.0 g of azobisisobutyronitrile (AIBN) was added, and the mixture was stirred at 50 °C to carry out prepolymerization for 4 hours to obtain a prepolymer. Then, 300 mL of n-heptane was added thereto (such that the difference in solubility parameter of the mixed solvent and the prepolymer was 0.1 MPa^{1/2}), and porous polymer V (71.6 g) was obtained in the same manner as in Example 1.

The measurements were performed in the same manner as in Example 1, and the various performance parameters of porous polymer V were shown in Table 2-2.

### (2) Preparation of porous polymer-nickel catalyst V

Under nitrogen atmosphere, porous polymer V (50.3 g, the amount of P was 150 mmol) and bis-(1,5-cyclooctadiene)nickel (8.3 g, 30 mmol) were added to 1500 mL of 3-pentenenitrile. Subsequently, catalyst V (52.1 g) was obtained in the same manner as in Example 1, and the content of Ni in catalyst V determined in the same manner as in Example 1 was shown in Table 2-2.

### (3) Preparation of adiponitrile

Preparation and analysis were carried out in the same manner as in Example 1 except that catalyst V was used. The analysis was carried out in the same manner as in Example 1, and the results were shown in Table 2-2.

### Example 26

### (1) Preparation of porous polymer W

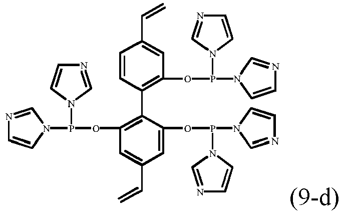

Monomer 9-d (44.8 g, 60 mmol) was dissolved in 600 mL of benzonitrile (the difference in solubility parameter of the solvent and the monomer was 1.39 MPa^{1/2}), 0.5 g of azobisisobutyronitrile (AIBN) was added, and the mixture was stirred at 50 °C to carry out prepolymerization for 4 hours to obtain a prepolymer. Then, 300 mL of n-heptane was added thereto (such that the difference in solubility parameter of the mixed solvent and the prepolymer was 0.1 MPa^{1/2}), and porous polymer W (41.0 g) was obtained in the same manner as in Example 1.

The measurements were performed in the same manner as in Example 1, and the various performance parameters of porous polymer W were shown in Table 2-2.

### (2) Preparation of porous polymer-nickel catalyst W

Under nitrogen atmosphere, porous polymer W (37.3 g, the amount of P was 150 mmol) and bis-(1,5-cyclooctadiene)nickel (8.3 g, 30 mmol) were added to 1500 mL of 3-pentenenitrile. Subsequently, catalyst W (39.1 g) was obtained in the same manner as in Example 1, and the content of Ni in catalyst W determined in the same manner as in Example 1 was shown in Table 2-2.

### (3) Preparation of adiponitrile

Preparation and analysis were carried out in the same manner as in Example 1 except that catalyst W was used. The analysis was carried out in the same manner as in Example 1, and the results were shown in Table 2-2.

### Example 27

### (1) Preparation of porous polymer X

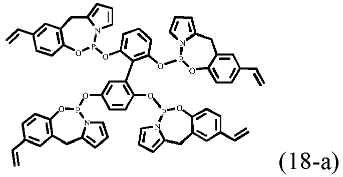

Monomer 18-a (67.6 g, 60 mmol) was dissolved in 750 mL of trichloromethane (the difference in solubility parameter of the solvent and the monomer was 1.15 MPa^{1/2}), 0.5 g of azobisisobutyronitrile (AIBN) was added thereto, and the mixture was stirred at 50 °C to carry out prepolymerization for 4 hours to obtain a prepolymer. Then, 187.5 mL of methyl propyl ketone was added thereto (such that the difference in solubility parameter of the mixed solvent and the prepolymer was 0.12 MPa^{1/2}), and porous polymer X (57.5 g) was obtained in the same manner as in Example 1.

The measurements were performed in the same manner as in Example 1, and the various performance parameters of porous polymer X were shown in Table 2-2.

### (2) Preparation of porous polymer-nickel catalyst X

Under nitrogen atmosphere, porous polymer X (42.3 g, the amount of P was 150 mmol) and bis-(1,5-cyclooctadiene)nickel (8.3 g, 30 mmol) were added to 1500 mL of 3-pentenenitrile. Subsequently, catalyst X (44.1 g) was obtained in the same manner as in Example 1, and the content of Ni in catalyst X determined in the same manner as in Example 1 was shown in Table 2-2.

### (3) Preparation of adiponitrile

Preparation and analysis were carried out in the same manner as in Example 1 except that catalyst X was used. The analysis was carried out in the same manner as in Example 1, and the results were shown in Table 2-2.

### Example 28

### (1) Preparation of porous polymer Y

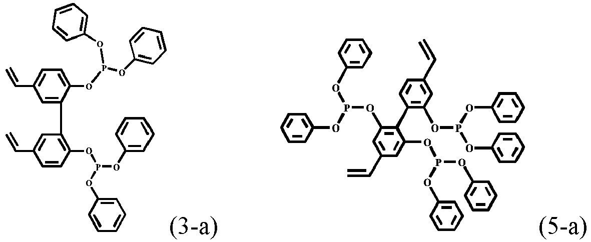

Monomer 3-a (40.2 g, 60 mmol) and monomer 5-a (54.1 g, 60 mmol) were dissolved in 1200 mL of 3-pentanone (the difference in solubility parameter of the solvent and the mixed monomer was 2.11 MPa^{1/2}), 1.0 g of azobisisobutyronitrile (AIBN) was added, and the mixture was stirred at 50 °C to carry out prepolymerization for 4 hours to obtain a prepolymer. Then, 300 mL of n-butyl acetate (such that the difference in solubility parameter of the mixed solvent and the prepolymer was 0.03 MPa^{1/2}), and porous polymer Y (82.0 g) was obtained in the same manner as in Example 1.

The measurements were performed in the same manner as in Example 1, and the various performance parameters of porous polymer Y were shown in Table 2-2.

### (2) Preparation of porous polymer-nickel catalyst Y

Under nitrogen atmosphere, porous polymer Y (47.2 g, the amount of P was 150 mmol) and bis-(1,5-cyclooctadiene)nickel (8.3 g, 30 mmol) were added to 1500 mL of 3-pentenenitrile. Subsequently, catalyst Y (48.9 g) was obtained in the same manner as in Example 1, and the content of Ni in catalyst Y determined in the same manner as in Example 1 was shown in Table 2-2.

### (3) Preparation of adiponitrile

Preparation and analysis were carried out in the same manner as in Example 1 except that catalyst Y was used. The analysis was carried out in the same manner as in Example 1, and the results were shown in Table 2-2.

### Example 29

### (1) Preparation of porous polymer Z

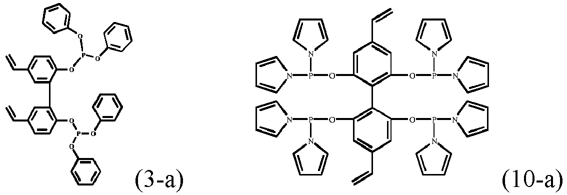

Monomer 3-a (40.2 g, 60 mmol) and monomer 10-a (55.1 g, 60 mmol) were dissolved in 1200 mL of ethyl acetate (the difference in solubility parameter of the solvent and the mixed monomer was 1.9 MPa^{1/2}), 1.0 g of azobisisobutyronitrile (AIBN) was added, and the mixture was stirred at 50 °C to carry out prepolymerization for 4 hours to obtain a prepolymer. Then, 300 mL of methyl isobutyl ketone was added thereto (such that the difference in solubility parameter of the mixed solvent and the prepolymer was 0.4 MPa^{1/2}), and porous polymer Z (85.7 g) was obtained in the same manner as in Example 1.

The measurements were performed in the same manner as in Example 1, and the various performance parameters of porous polymer Z were shown in Table 2-2.

### (2) Preparation of porous polymer-nickel catalyst Z

Under nitrogen atmosphere, porous polymer Z (39.7 g, the amount of P was 150 mmol) and bis-(1,5-cyclooctadiene)nickel (8.3 g, 30 mmol) were added to 1500 mL of 3-pentenenitrile. Subsequently, catalyst Z (41.5 g) was obtained in the same manner as in Example 1, and the content of Ni in catalyst Z determined in the same manner as in Example 1 was shown in Table 2-2.

### (3) Preparation of adiponitrile

Preparation and analysis were carried out in the same manner as in Example 1 except that catalyst Z was used. The analysis was carried out in the same manner as in Example 1, and the results were shown in Table 2-2.

### Example 30

### (1) Preparation of porous polymer A1 1

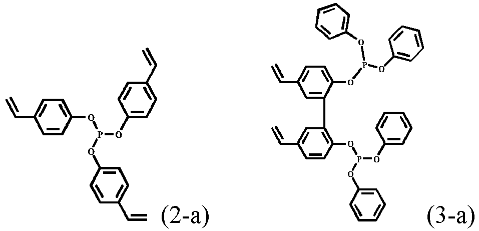

Monomer 2-a (23.3 g, 60 mmol) and monomer 3-a (13.4 g, 20 mmol) were dissolved in 600 mL of tetrahydrofuran (the difference in solubility parameter of the solvent and the mixed monomer was 2.38 MPa^{1/2}), 0.7 g of azobisisobutyronitrile (AIBN) was added, and the mixture was stirred at 50 °C to carry out prepolymerization for 4 hours to obtain a prepolymer. Then, 100 mL of n-heptane was added thereto (such that the difference in solubility parameter of the mixed solvent and the prepolymer was 0.4 MPa^{1/2}), and porous polymer A11 (33.0 g) was obtained in the same manner as in Example 1.

The measurements were performed in the same manner as in Example 1, and the various performance parameters of porous polymer A11 were shown in Table 2-2.

### (2) Preparation of porous polymer-nickel catalyst A11

Under nitrogen atmosphere, porous polymer A11 (31.7 g, the amount of P was 90 mmol) and bis-(1,5-cyclooctadiene)nickel (5.0 g, 18 mmol) were added to 1500 mL of 3-pentenenitrile. Subsequently, catalyst A11 (32.8 g) was obtained in the same manner as in Example 1, and the content of Ni in catalyst A11 determined in the same manner as in Example 1 was shown in Table 2-2.

### (3) Preparation of adiponitrile

Preparation and analysis were carried out in the same manner as in Example 1 except that catalyst A11 was used. The analysis was carried out in the same manner as in Example 1, and the results were shown in Table 2-2.

### Example 31

### (1) Preparation of porous polymer A12

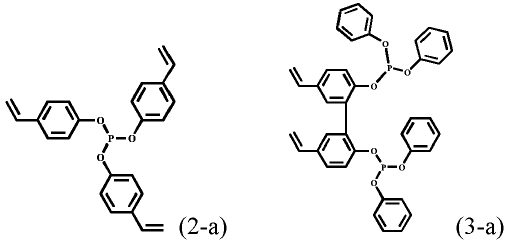

Monomer 2-a (23.3 g, 60 mmol) and monomer 3-a (402.1 g, 600 mmol) were dissolved in 8000 mL of tetrahydrofuran (the difference in solubility parameter of the solvent and the mixed monomer was 2.3 MPa^{1/2}), 10 g of azobisisobutyronitrile (AIBN) was added, and the mixture was stirred at 50 °C to carry out prepolymerization for 4 hours to obtain a prepolymer. Then, 1330 mL of n-heptane was added thereto (such that the difference in solubility parameter of the mixed solvent and the prepolymer was 0.14 MPa^{1/2}), porous polymer A12 (387.1 g) was obtained in the same manner as in Example 1.

The measurements were performed in the same manner as in Example 1, and the various performance parameters of porous polymer A12 were shown in Table 2-2.

### (2) Preparation of porous polymer-nickel catalystA12

Under nitrogen atmosphere, porous polymer A12 (52.9 g, the amount of P was 150 mmol) and bis-(1,5-cyclooctadiene)nickel (8.3 g, 30 mmol) were added to 1500 mL of 3-pentenenitrile. Subsequently, catalyst A12 (54.7 g) was obtained in the same manner as in Example 1, and the content of Ni in catalyst A12 determined in the same manner as in Example 1 was shown in Table 2-2.

### (3) Preparation of adiponitrile

Preparation and analysis were carried out in the same manner as in Example 1 except that catalyst A12 was used. The analysis was carried out in the same manner as in Example 1, and the results were shown in Table 2-2.

### Example 32

### (1) Preparation of porous polymer A13

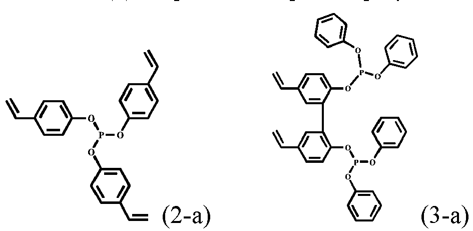

Monomer 2-a (2.33 g, 6 mmol) and monomer 3-a (80.42 g, 120 mmol) were dissolved in 1600 mL of tetrahydrofuran (the difference in solubility parameter of the solvent and the mixed monomer was 2.3 MPa^{1/2}), and 2.0 g of azobisisobutyronitrile (AIBN) was continously added, and the mixture was stirred at 50 °C to carry out prepolymerization for 4 hours to obtain a prepolymer. Then, 266 mL of n-heptane was added thereto (such that the difference in solubility parameter of the mixed solvent and the prepolymer was 0.12 MPa^{1/2}), and porous polymer A13 (73.6 g) was obtained in the same manner as in Example 1.

The measurements were performed in the same manner as in Example 1, and the various performance parameters of porous polymer A13 were shown in Table 2-2.

### (2) Preparation of porous polymer-nickel catalyst A13

Under nitrogen atmosphere, porous polymer A13 (52.9 g, the amount of P was 150 mmol) and bis-(1,5-cyclooctadiene)nickel (8.3 g, 30 mmol) were added to 1500 mL of 3-pentenenitrile. Subsequently, catalyst A13 (54.7 g) was obtained in the same manner as in Example 1, and the content of Ni in catalyst A13 determined in the same manner as in Example 1 was shown in Table 2-2.

### (3) Preparation of adiponitrile

Preparation and analysis were carried out in the same manner as in Example 1 except that the catalyst A13 was used. The analysis was carried out in the same manner as in Example 1, and the results were shown in Table 2-2.

### Example 33

### (1) Preparation of porous polymer A14

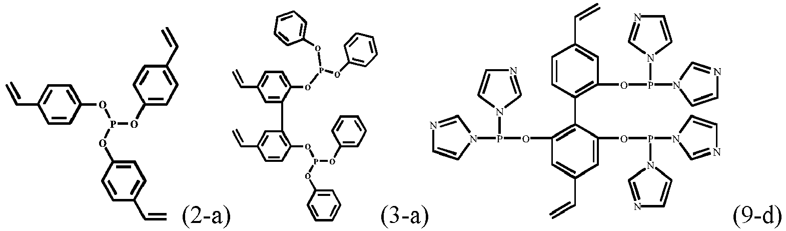

Monomer 2-a (23.3 g, 60 mmol), monomer 3-a (40.2 g, 60 mmol), and monomer 9-d (44.8 g, 60 mmol) were dissolved in 1600 mL of tetrahydrofuran (the difference in solubility parameter of the solvent and the mixed monomer was 2.1 MPa^{1/2}), 2.0 g of azobisisobutyronitrile (AIBN) was added, and the mixture was stirred at 50 °C to carry out prepolymerization for 4 hours to obtain a prepolymer. Then, 400 mL of n-heptane was added thereto (such that the difference in solubility parameter of the mixed solvent and the prepolymer was 0.18 MPa^{1/2}), and porous polymer A14 (96.39 g) was obtained in the same manner as in Example 1.

The measurements were performed in the same manner as in Example 1, and the performance parameters of porous polymer A14 were shown in Table 2-2.

### (2) Preparation of porous polymer-nickel catalyst A14

Under nitrogen atmosphere, porous polymer A14 (56.15 g, the amount of P was 180 mmol) and bis-(1,5-cyclooctadiene)nickel (8.3 g, 30 mmol) were added to 1500 mL of 3-pentenenitrile. Subsequently, catalyst A13 (58.7 g) was obtained in the same manner as in Example 1, and the content of Ni in catalyst A13 determined in the same manner as in Example 1 was shown in Table 2-2.

### (3) Preparation of adiponitrile

Preparation and analysis were carried out in the same manner as in Example 1 except that the catalyst A13 was used. The analysis was carried out in the same manner as in Example 1, and the results were shown in Table 2-2.

### Example 34

### (1) Preparation of porous polymer U1

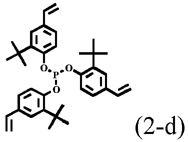

Monomer (2-d) (100.1 g, 180 mmol) was dissolved in 1200 mL of 1,2-dichloroethane (the difference in solubility parameter of the solvent and the monomer was 2.09 MPa^{1/2}), 1.5g of azobisisobutyronitrile (AIBN) was added, and the mixture was stirred at 50 °C to carry out prepolymerization for 4 hours to obtain a prepolymer. Then, 600 mL of benzonitrile was added thereto (such that the difference in solubility parameter of the mixed solvent and the prepolymer was 0.31 MPa^{1/2}), and porous polymer U1 (80.1 g) was obtained in the same manner as in Example 1.

The measurements were performed in the same manner as in Example 1, and the various performance parameters of porous polymer U1 were shown in Table 2-2.

### (2) Preparation of porous polymer-nickel catalyst U1

Under nitrogen atmosphere, porous polymer U1 (13.91 g, the amount of P was 25 mmol) and bis-(1,5-cyclooctadiene)nickel (1.38 g, 5 mmol) were added to 250 mL of 3-pentenenitrile. Subsequently, catalyst U1 (12.78 g) was obtained in the same manner as in Example 1, and the content of Ni in catalyst U1 determined in the same manner as in Example 1 was shown in Table 2-2.

### (3) Preparation of adiponitrile

Preparation and analysis were carried out in the same manner as in Example 1 except that catalyst U1 was used. The analysis was carried out in the same manner as in Example 1, and the results were shown in Table 2-2.

### Comparative Example 1

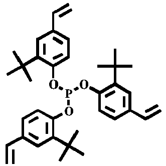

The monomer (100.1 g, 180 mmol) was dissolved in 1001 ml of tetrahydrofuran, and 2.5 g of azobisisobutyronitrile (AIBN) was added. The obtained mixed solution was transferred into an autoclave, and reacted at 100 °C for 24 hours. After the reaction was completed, the solid product was rinsed with dichloromethane and vacuum dried to obtain porous polymer b (64.1 g). After characterizing by BET test, the specific surface area, the pore volume of 1.20 cm³/g, the ratio of the pore volume of pores with pore diameters of < 10 nm and > 15 nm, and the P content of the porous polymer b obtained were shown in Table 2-2.

### (2) Preparation of porous polymer-nickel catalyst b

Under nitrogen atmosphere, porous polymer b (14.56 g, the amount of P was 25 mmol) and bis-(1,5-cyclooctadiene)nickel (1.38 g, 5 mmol) were added to 250 mL of 3-pentenenitrile. Subsequently, catalyst b (12.68 g) was obtained in the same manner as in Example 1, and the content of Ni in catalyst b determined in the same manner as in Example 1 was shown in Table 2-2.

### (3) Preparation of adiponitrile

Preparation and analysis were carried out in the same manner as in Example 1 except that the catalyst b was used. The analysis was carried out in the same manner as in Example 1, and the results were shown in Table 2-2.

The results of the above measurements of Examples 1 to 34 and Comparative Example 1 were listed in Tables 2-1 and 2-2 below.

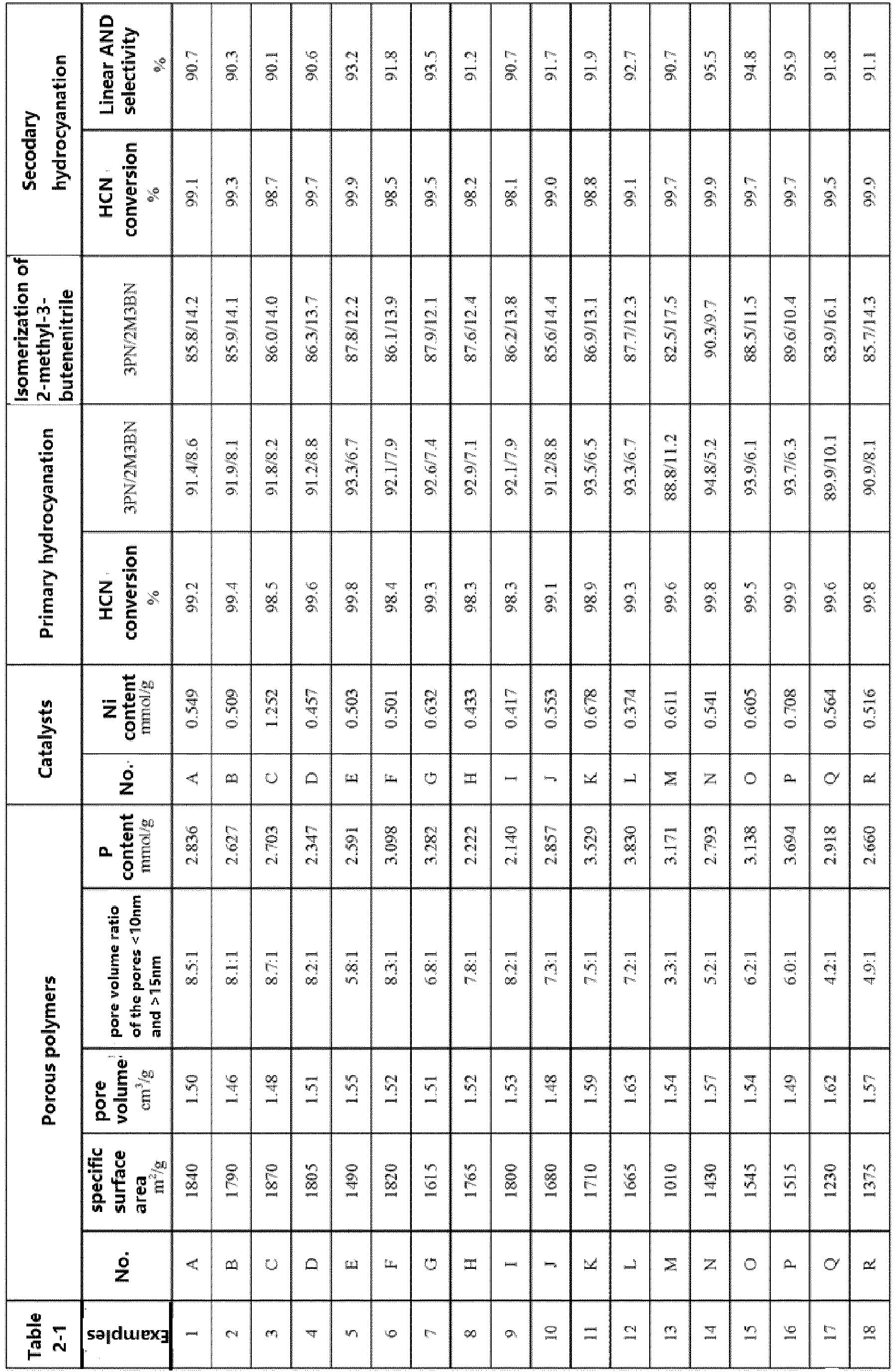

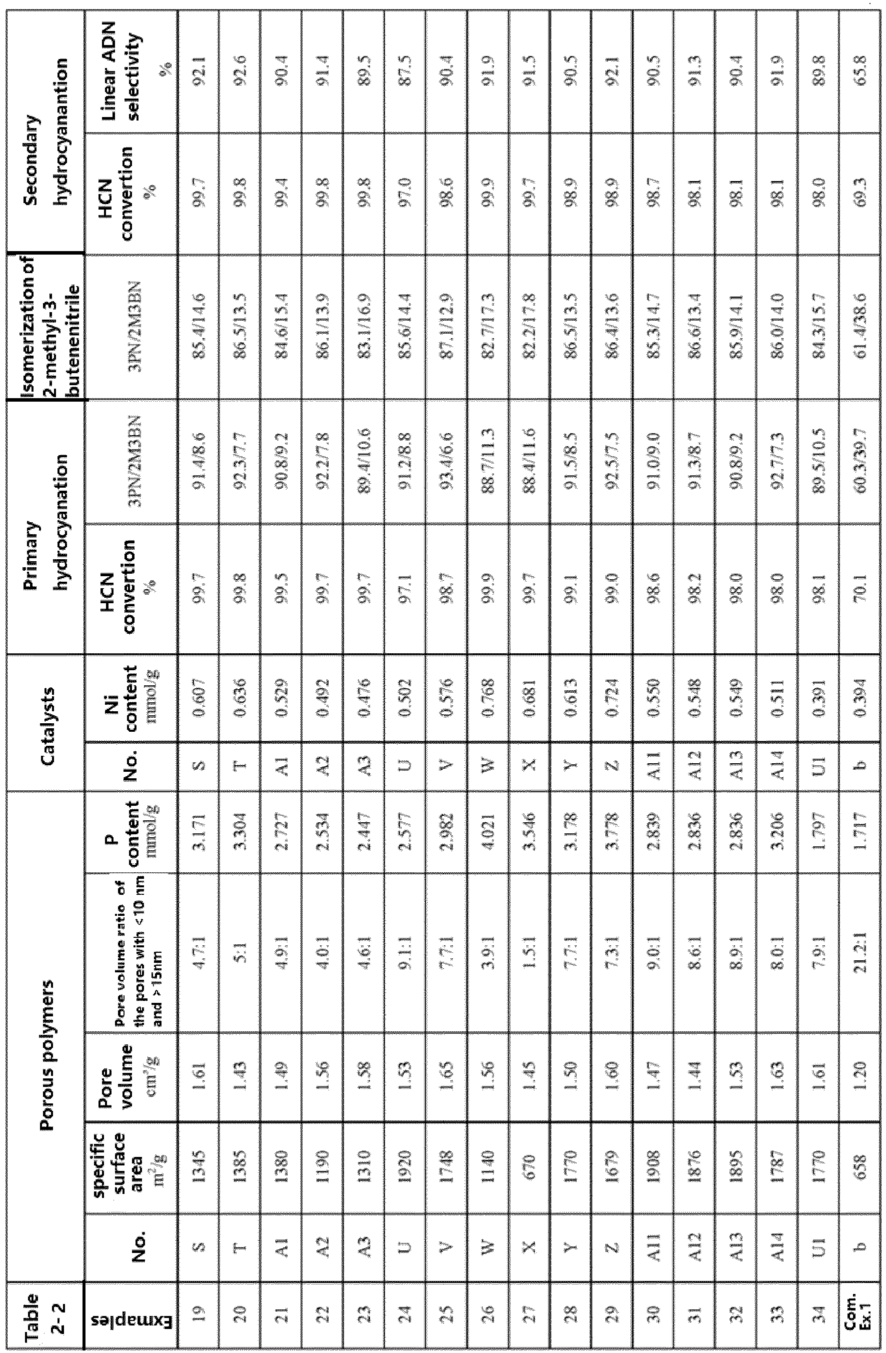

As can be seen from the above Tables 2-1 and 2-2, the porous polymers obtained by the method for preparing the porous polymer of the present disclosure in Examples 1 to 34 have a specific range of pore volume ratios of secondary pore channels, and the porous polymer-nickel catalyst obtained from the porous polymer that meets all technical features of the present application has excellent catalytic activity, high reaction selectivity, and high product linearity. The selectivity of linear ADN is 87.5% or more, and the optimal selectivity reached 95.9%. Furthermore, it can be seen from the above examples that the pore volume ratio and specific surface area of the secondary pore channels, the ratio between the phosphorus ligands in the copolymer, the charge on the phosphorus atom, and the steric hindrance of the phosphorus ligands and so on will all affect the reactivity and the linearity, only the best combination may achieve the best effect. Example 16 represents the better combination.

In Example 34 of the present disclosure compared with Comparative example 1, it can be seen that compared with the parameters in Example 34 of the present application and Comparative Example 1, the specific surface area, pore volume ratio, and P content are relatively close. However, the pore volume ratio of the pores with < 10 nm and > 15 nm is 7.9:1 in Example 34, while the ratio of the pore volume of the pores with < 10 nm and > 15 nm of the porous polymer prepared by the method of solvothermal polymerization in Comparative Example 1 is 21.2: 1, which is not within the scope of this application. Therefore, when the porous polymer-nickel catalyst obtained from Comparative Example 1 was used for the preparation of adiponitrile, both the reaction selectivity and the linear ADN selectivity were lower.

### Example 35

### Catalyst A of Example 1 was reacted with different water content systems as follows:

Catalyst A (the content of Ni was 10 mmol) prepared in Example 1 was used, according to the same method for preparing adiponitrile as in Example 1, the reaction was carried out in systems with different water contents (100ppm, 500ppm, 1000ppm, 2000ppm), respectively, and the composition content of the product was analyzed by GC. The specific experimental results were shown in Table 3.

**Table 3**

| Group No. | Water content /ppm | Primary hydrocyanation | | Isomerization of 2-methyl-3-but enenitrile | Secondary hydrocyanation | |
|---|---|---|---|---|---|---|
| | | HCN conversion % | 3 PN/2M3 BN | 3 PN/2M3 BN | HCN conversion % | Linear ADN selectivity% |
| 1 | 100 | 99.4 | 92.6/7.4 | 86.2/13.8 | 99.3 | 90.5 |
| 2 | 500 | 99.4 | 92.3/7.7 | 86.0/14.0 | 99.3 | 90.7 |
| 3 | 1000 | 99.3 | 92.2/7.8 | 85.9/14.1 | 99.1 | 90.6 |
| 4 | 2000 | 99.1 | 92.1/7.9 | 85.9/14.1 | 99.0 | 90.7 |

### Comparative Example 2

The phosphorus ligand monomer used in Example 1 was used to prepare catalyst a, and the performance of catalyst a was evaluated in different water content systems as follows:

### (1) Preparation of catalyst a

Under nitrogen atmosphere, equimolar amounts (60 mmol) of monomer 2-a and monomer 3-a and bis-(1,5-cyclooctadiene)nickel (17 g, 70 mmol) were added to 1500 mL of 3-pentenenitrile, followed by continued stirring at room temperature for 24 hours. After the stirring was completed, the solvent was removed under reduced pressure in vacuum to give catalyst a (78.1 g). The content of Ni in catalyst a determined by inductively coupled plasma optical emission spectrometer (ICP-OES) was 0.895 mmol/g.

### (2) Evaluation of the performance of catalyst a in different water content systems

Catalyst a (the content of Ni was 10 mmol) was used, according to the same method for preparing adiponitrile as in Example 1, the reaction was carried out in systems with different water contents (100ppm, 500ppm, 1000ppm, 2000ppm), respectively, the sampling and filteration were performed, and the composition content of the product was analyzed by GC. The specific experimental results were shown in Table 4.

**Table 4**

| Group No. | Water content /ppm | Primary hydrocyanation | | Isomerization of 2-methyl-3-but enenitrile | Secondary hydrocyanation | |
|---|---|---|---|---|---|---|
| | | HCN conversion % | 3 PN/2M3 BN | 3 PN/2M3 BN | HCN conversion % | Linear ADN selectivity % |
| 1 | 100 | 99.5 | 91.3/8.7 | 83.7/16.3 | 99.6 | 89.6 |
| 2 | 500 | 86.3 | 84.3/15.7 | 71.0/39.0 | 88.2 | 89.4 |
| 3 | 1000 | 72.1 | 69.4/20.6 | 49.2/51.8 | 69.8 | 89.6 |
| 4 | 2000 | 45.3 | 46.1/53.9 | 36.6/63.4 | 39.7 | 89.3 |

As can be seen from Tables 3 and 4 above, as compared Example 35 of the present disclosure using the catalyst made from the porous polymer of the present disclosure with Comparative Example 2 using the catalyst made from the phosphorus ligand that had not been polymerized, the catalyst of the present disclosure has excellent water resistance, so that the separation and recovery steps of the catalyst may be simplified, and the recycling times of the catalyst may be greatly increased.

### Example 36

Catalyst A (Ni 0.549 mmol/g) prepared in Example 1 was used, the reaction was carried out according to the same method for preparing adiponitrile as in Example 1, and the composition content of the product was analyzed by GC. The content of Ni in the catalyst was determined by inductively coupled plasma optical emission spectrometer (ICP-OES). After the reaction was completed, the catalyst A was separated and recovered from the reaction liquid by filtration. Further, after washing, the catalyst was used for subsequent recycling, and the recycling was repeated 30 times. The product composition content was respectively measured after not recycling, recycling for the 10th time, recycling for the 20th time, and recycling for the 30th time. The content of Ni in the catalyst was measured after not recycling and recycling for the 30th time. The results were shown in Table 5.

**Table 5**

| Group No. | Recycling times | Primary hydrocyanation | | | Isomerization of 2-methyl-3 -butenenitrile | | Hydrocyanation of 3-pentenenitrile | | |
|---|---|---|---|---|---|---|---|---|---|
| | | Ni content in catalyst A mmol/g | HCN conversion % | 3PN/ 2M3B N | Ni content in catalyst A mmol/g | 3PN/ 2M3BN | Ni content in catalyst A mmol/g | HCN conversion % | Linear ADN selectivit y% |
| 1 | 0 | 0.549 | 99.4 | 92.6/7 .4 | 0.549 | 85.5/14.5 | 0.549 | 99.3 | 90.7 |
| 2 | 10 | / | 99.3 | 92.4/7 .6 | / | 85.3/14.7 | / | 99.2 | 90.6 |
| 3 | 20 | / | 99.3 | 92.5/7 .5 | / | 85.2/14.8 | / | 99.1 | 90.5 |
| 4 | 30 | 0.541 | 99.2 | 92.5/7 .5 | 0.542 | 85.2/14.8 | 0.545 | 99.1 | 90.5 |

### Example 37

Catalyst A (Ni 0.549 mmol/g) prepared in Example 1 was used, except that the method for preparing adiponitrile in Example 35 was changed to a continuous reaction mode, and a precision filtration device was installed at the discharge port of the continuous reaction so that catalyst A was guaranteed to remain in the reaction system. The measurements and evaluations were performed in the same manner as in Example 35 except operated for 0 hours, 100 hours, 150 hours, and 200 hours, respectively, and the results were shown in Table 6.

**Table 6**

| Group No. | Stable running time/h | Primary hydrocyanation | | | Isomerization of 2-methyl-3 -butenenitrile | | Secondary hydrocyanation | | |
|---|---|---|---|---|---|---|---|---|---|
| | | Ni content in catalyst A mmol/g | HCN conversion % | 3PN/ 2M3BN | Ni content in catalyst A mmol/g | 3PN/ 2M3BN | Ni content in catalyst A mmol/g | HCN conversi on % | ADN selectivity % |
| 1 | 0 | 0.549 | 99.4 | 92.6/7.4 | 0.549 | 85.5/14.5 | 0.549 | 99.3 | 90.7 |
| 2 | 100 | / | 99.2 | 92.5/7.5 | / | 85.3/14.7 | / | 99.1 | 90.6 |
| 3 | 150 | / | 99.1 | 92.4/7.6 | / | 85.1/14.9 | / | 98.9 | 90.6 |
| 4 | 200 | 0.539 | 99.1 | 92.5/7.5 | 0.541 | 85.2/14.8 | 0.543 | 98.8 | 90.5 |

It can be seen from the above Examples 36 and 37 and Tables 5 and 6 that the porous polymer-nickel catalyst made of the porous polymer of the present disclosure may be used for multiple cycles of recycling and stable operation for a long time, and still maintain excellent catalytic activity in the process of multiple recycling.

## Claims

1. A porous polymer containing a phosphorous ligand, wherein the porous polymer has a pore volume of 0.3 to 2.5 cm³/g, preferably 0.5 to 2.0 cm³/g;
the porous polymer comprises a pore having a first pore diameter and a pore having a second pore diameter, and a ratio of a pore volume of the pore having a first pore diameter to a pore volume of the pore having a second pore diameter is 1 to 10:1, preferably 2 to 8:1,
the pore having a first pore diameter has a pore diameter of less than 10 nm, preferably 2 to 6 nm, as measured by a nitrogen adsorption method using an NLDFT model; the pore having a second pore diameter has a pore diameter of greater than 15 nm, preferably greater than 20 nm, as measured by a nitrogen adsorption method using an NLDFT model; and
the porous polymer is obtained by self-polymerization or copolymerization of at least one of the phosphorus ligands, and a phosphorous content of the porous polymer is 1 to 5 mmol/g, preferably 1.7 to 3.9 mmol/g.

2. The porous polymer according to claim 1, wherein a BET specific surface area of the porous polymer is 100 to 2000m²/g, preferably 500 to 1700m²/g.

3. The porous polymer according to claim 1 or 2, the phosphorous ligand is represented by the following general formula (1): 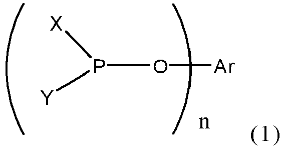
wherein : n = 1 to 4;
Ar represents a group having a substituted aromatic ring structure;
X and Y are the same or different, and each independently represents an aryloxy group or a nitrogen-containing heterocyclic group, and X and Y form a ring via a single bond or a methylene group.

4. The porous polymer according to claim 3, in general formula (1):
when n is 1, the phosphorous ligand is a monodentate phosphorous ligand, and both X and
Y are 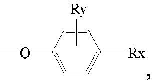 Ar is 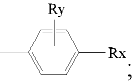 when n is 2 to 4, the phosphorous ligand is a multidentate phosphorous ligand, X and Y are the same or different, and each independently represents 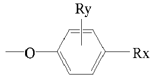 or a nitrogen-containing heterocyclic group; Ar is 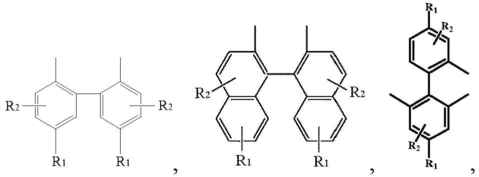 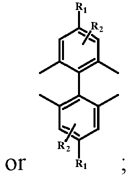 provided that, when both X and Y represent 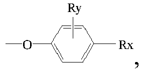 X and Y may not form a ring; when both X and Y represent a nitrogen-containing heterocyclic group, X and Y may not form a ring or form a ring via a single bond or a methylene group; and when X is 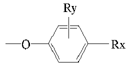 and Y is a nitrogen-containing heterocyclic group, X and Y form a ring via a methylene group;
the nitrogen-containing heterocyclic group is 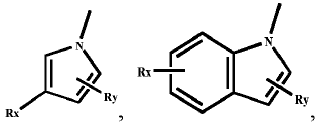 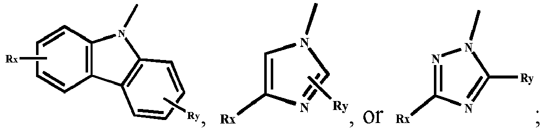
among the above, R₁ is selected from the group consisting of a hydrogen atom, a vinyl group, a propenyl group, an acryloyl group, an acrylate group, or a methacryloyl group;
R₂ is selected from the group consisting of a hydrogen atom, a halogen atom, a nitrile group, an C₁-C₁₀ alkyl group, an C₁-C₁₀ alkoxy group, an C₁-C₁₀ alkanoyl group, an C₁-C₁₀ ester group, or an C₁-C₁₀ sulfonate group;
Rₓ is selected from the group consisting of a hydrogen atom, a vinyl group, a propenyl group, an acryloyl group, an acrylate group, or a methacryloyl group;
R_{y} is selected from the group consisting of a hydrogen atom, a halogen atom, a nitrile group, an C₁-C₁₀ alkyl group, an C₁-C₁₀ alkoxy group, an C₁-C₁₀ alkanoyl group, an C₁-C₁₀ ester group, or an C₁-C₁₀ sulfonate group.

5. The porous polymer according to any of claims 1 to 4, wherein the phosphorous ligand is selected from the compounds having the following structural formulae (2) to (18): 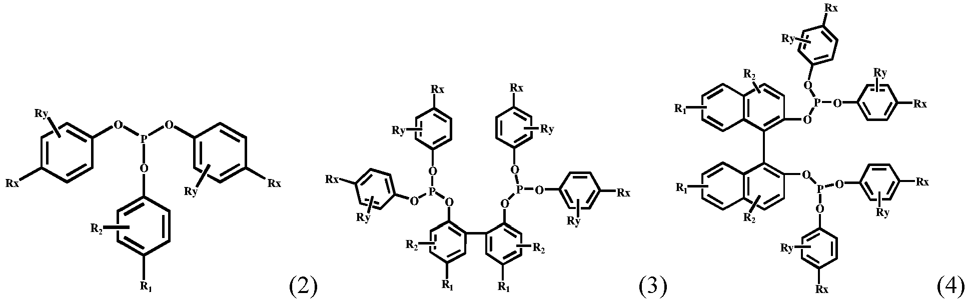 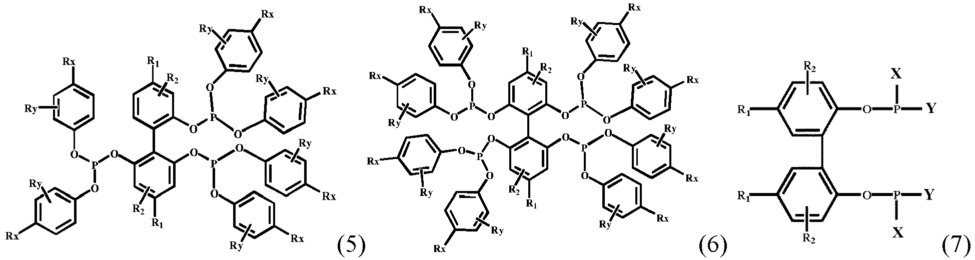 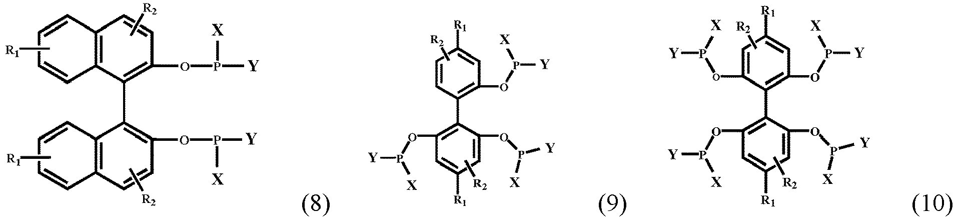 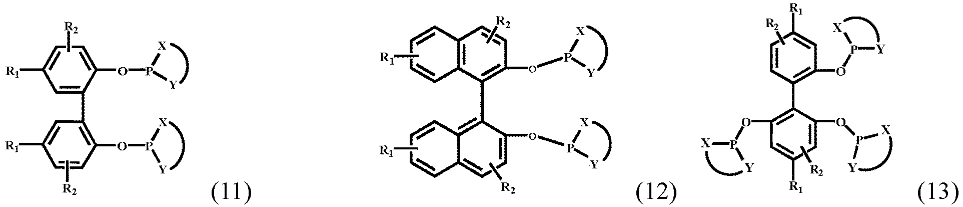 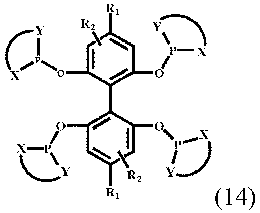 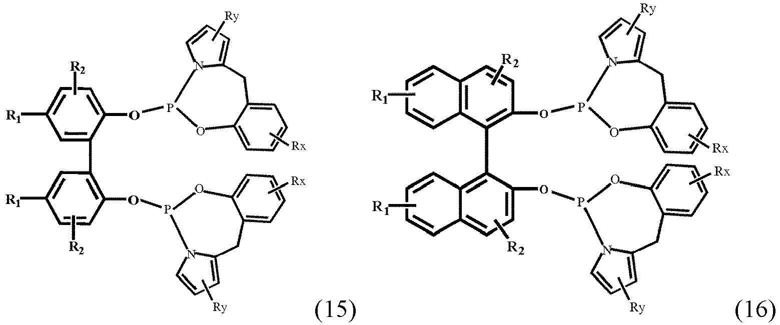 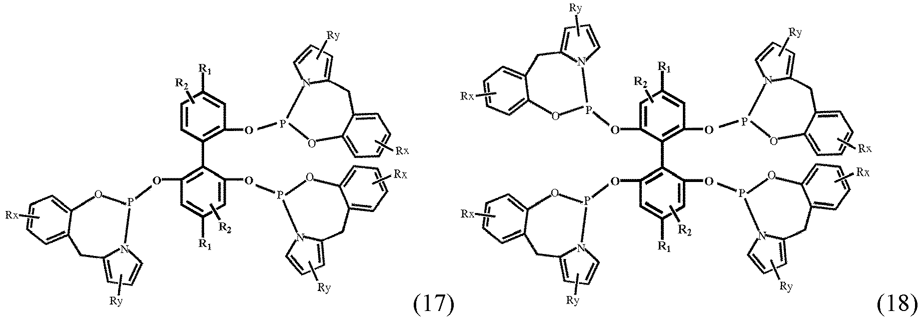 wherein R₁, R₂, Rₓ, and R_{y} are as defined in general formula (1); X and Y are the same, which both represent a nitrogen-containing heterocyclic group.

6. The porous polymer according to claim 4 or 5, among the phosphorous ligands, both X and Y are nitrogen-containing heterocyclic groups, and a structural moiety at which X and Y form a ring via a single bond or a methylene group is selected from any one of the group consisting of the following:

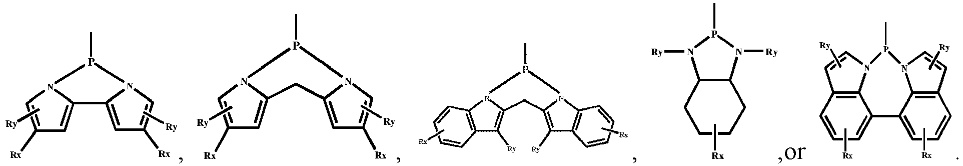

7. The porous polymer according to any of claims 1 to 6, wherein the porous polymer is obtained by self-polymerization of any one of the phosphorus ligands.

8. The porous polymer according to any of claims 1 to 6, wherein the porous polymer is a random copolymer obtained by copolymerization of any two of the phosphorus ligands, and a molar ratio between the two phosphorous ligands is 0.01 to 3:1, preferably 0.05 to 2.5:1.

9. The porous polymer according to any of claims 1 to 6, wherein the porous polymer is a random copolymer obtained by copolymerization of any three or more of the phosphorous ligands.

10. A method for preparing the porous polymer containing a phosphorous ligand according to any of claims 1 to 9, wherein at least one of the phosphorous ligands is self-polymerized or copolymerized in the presence of a radical initiator.

11. The method according to claim 10, wherein the method comprises: subjecting at least one of the phosphorous ligands to prepolymerization in the presence of a first organic solvent to obtain a prepolymer, wherein the difference in solubility parameter of at least one of the phosphorous ligands and the first organic solvent is 1.0 to 2.5[MPa]^{1/2}; adding a second organic solvent to the resulting prepolymer such that the difference in solubility parameter of a mixed solvent of the first and second organic solvents and the prepolymer is less than 0.5 [MPa]^{1/2}, and swelling and curing the prepolymer.

12. The method according to claim 11, wherein the prepolymerization temperature is 50 to 80 °C, and the prepolymerization time is 2 to 10 hours; the swelling and curing temperature is 85 to 110 °C, and the swelling and curing time is 2 to 10 hours.

13. The method according to any of claims 10 to 12, wherein any one of the phosphorous ligand is self-polymerized.

14. The method according to any of claims 10 to 12, wherein any two of the phosphorous ligands are copolymerized, and the molar ratio between the two phosphorous ligands is 0.01 to 3:1, preferably 0.05 to 2.5:1.

15. The method according to any of claims 10 to 12, wherein any three or more of the phosphorous ligands are copolymerized.

16. The method according to any of claims 10 to 15, wherein the radical initiator is selected from at least one of the group consisting of 2,2'-azobisisobutyronitrile and 2,2'-azobis(2-methylpropionitrile).

17. The method according to any of claims 11 to 16, wherein the first organic solvent and the second organic solvent are the same or different, and either of them is one or more selected from the group consisting of n-pentane, n-hexane, n-heptane, dodecane, cyclohexane, isobutyl acetate, benzonitrile, methyl isobutyl ketone, n-butyl acetate, cyclopentane, 3-pentanone, p-xylene, toluene, methyl propyl ketone, tetrahydrofuran, ethyl acetate, benzene, trichloromethane, 1,1,2-trichloroethane, methyl acetate, 1,2-dichloroethane, acetone, cyclohexanone, 1,4-dioxane, cyclopentanone, propionitrile, ethanol, dimethyl sulfoxide, methanol, and water.

18. A porous polymer-nickel catalyst, comprising the porous polymer containing a phosphorous ligand according to any of claims 1 to 9 and a zero-valent nickel, and the content of the zero-valent nickel is 0.1 to 2 mmol/g with respect to an amount of the catalyst.

19. A method for preparing adiponitrile, wherein the method comprises: sequentially subjecting to a primary hydrocyanation reaction of butadiene, an isomerization reaction of branched mononitriles, and a secondary hydrocyanation reaction of linear mononitriles in the presence of the porous polymer-nickel catalyst according to claim 18.

20. The method according to claim 19, wherein the method comprises the following steps of
(1) primary hydrocyanation reaction
subjecting butadiene and hydrocyanic acid to a primary hydrocyanation reaction in the presence of the catalyst, wherein the molar ratio of the butadiene to the hydrocyanic acid is 1.0 to 1.5, and the ratio of a mole number of hydrocyanic acid to a mole number of the catalyst in terms of zero-valent nickel is 100 to 1: 1, preferably 70 to 10:1, and a reaction temperature is 60 to 140 °C, and a reaction pressure is 0.3 to 5.0 MPa;
(2) isomerization reaction of branched mononitriles
subjecting a mixture of branched mononitrile separated from a product obtained in step 1 to an isomerization reaction in the presence of the catalyst, wherein the ratio of the mole number of the mixture of branched mononitriles to the mole number of the catalyst in terms of zero-valent nickel is 300 to 20: 1, preferably 200 to 50: 1, and the reaction temperature is 80 to 170 °C, and the reaction pressure is 0.3 to 5.0 MPa;
(3) secondary hydrocyanation reaction
subjecting a mixture of linear mononitriles separated from the products obtained in steps (1) and (2) and hydrocyanic acid to a secondary hydrocyanation reaction in the presence of the catalyst, wherein the molar ratio of the mixture of linear mononitriles to the hydrocyanic acid is 1.0 to 1.5, the ratio of the mole number of the hydrocyanic acid to the mole number of the catalyst in terms of zero-valent nickel is 1000 to 20: 1, preferably 500 to 20:1, and the reaction temperature is 30 to 120 °C, and the reaction pressure is 0.3 to 5.0 MPa.

21. The method according to claim 20, wherein the secondary hydrocyanation reaction is performed in the presence of a promoter, and the ratio of the mole number of the promoter to the mole number of the catalyst in terms of zero-valent nickel is 0.05 to 2.5: 1; and the promoter is a Lewis acid.

22. The method according to any of claims 19 to 21, wherein the method further comprises recycling the porous polymer-nickel catalyst, and the recycling comprises the following method (a) or (b):
(a) discharging at least part of the porous polymer-nickel catalyst together with a reaction product, filtering and separating, washing, and recycling in the next batch of reactions; or
(b) discharging at least part of the porous polymer-nickel catalyst without the reaction product, and recycling the catalyst still remain the reaction system.
